(19) Europäisches Patentamt
European Patent Office
Office européen des brevets

(11) **EP 1 605 979 B1**

(12) **EUROPEAN PATENT SPECIFICATION**

(45) Date of publication and mention
of the grant of the patent:
**22.10.2008 Bulletin 2008/43**

(51) Int Cl.:
***A61K 51/08*** (2006.01)     ***A61K 49/08*** (2006.01)

(21) Application number: **04711636.3**

(86) International application number:
**PCT/GB2004/000582**

(22) Date of filing: **17.02.2004**

(87) International publication number:
**WO 2004/071536 (26.08.2004 Gazette 2004/35)**

(54) **CONJUGATES FOR MEDICAL IMAGING COMPRISING CARRIER, TARGETING MOIETY AND A CONTRAST AGENT**

KONJUGATE FÜR MEDIZINISCHE ABBILDUNGEN UMFASSEND TRÄGER, TARGETING-ANTEILE UND KONTRASTMITTEL

CONJUGUES DESTINES A L'IMAGERIE MEDICALE ET COMPRENANT UN VEHICULE, UNE FRACTION DE CIBLAGE ET UN AGENT DE CONTRASTE

(84) Designated Contracting States:
**AT BE BG CH CY CZ DE DK EE ES FI FR GB GR HU IE IT LI LU MC NL PT RO SE SI SK TR**

(30) Priority: **17.02.2003 GB 0303532**
**17.02.2003 GB 0303533**

(43) Date of publication of application:
**21.12.2005 Bulletin 2005/51**

(73) Proprietors:
- **Upperton Limited**
**Nottingham NG2 5NA (GB)**
- **Novozymes Biopharma UK Limited**
**Nottingham NG7 1FD (GB)**

(72) Inventor: **WOODROW, John Rodney**
**West Bridgford,**
**Nottingham NG2 6AY (GB)**

(74) Representative: **Jones, Stephen Anthony et al**
**AdamsonJones**
**BioCity Nottingham**
**Pennyfoot Street**
**Nottingham NG1 1GF (GB)**

(56) References cited:
**US-A- 5 364 613**          **US-A- 5 582 172**

- **MANABE Y ET AL: "HIGH-LEVEL CONJUGATION OF CHELATING AGENTS INTO IMMUNOGLOBULINS USE OF AN INTERMEDIARY POLY-L-LYSINE-DIETHYLENETRIAMINEPENTAACETI C-ACID CARRIER" BIOCHIMICA ET BIOPHYSICA ACTA, vol. 883, no. 3, 1986, pages 460-467, XP009030737 ISSN: 0006-3002**
- **TORCHILIN V P ET AL: "MONOCLONAL ANTIBODY MODIFICATION WITH CHELATE-LINKED HITGH-MOLECULAR-WEIGHT POLYMERS: MAJOR INCREASES IN POLYVALENT CATION BINDING WITHOUT LOSS OF ANTIGEN BINDING" HYBRIDOMA, LIEBERT, NEW YORK, NY, US, vol. 6, no. 3, June 1997 (1997-06), pages 229-240, XP001069251 ISSN: 0272-457X**

**Description**

FIELD OF THE INVENTION

This invention relates to the delivery of agents to the body. One particular class of such agents are contrast agents useful in medical imaging techniques. The agents may be metals useful as contrast agents in magnetic resonance imaging (MRI), or in nuclear imaging, including positron emission tomography (PET), or as therapeutic agents in radiotherapy. The agents may alternatively be contrast agents useful in X-ray imaging. The invention also relates to methods by which agents for delivery to the body can be coupled to carriers and to targeting moieties effective to direct the agent to a specific locus within the body.

BACKGROUND OF THE INVENTION

It is known to enhance the contrast of images obtained by techniques such as MRI or X-ray imaging, by the prior administration of suitable contrast agents. In the case of X-ray imaging such agents are typically highly radio-opaque materials, while for MRI they are typically paramagnetic species that affect the relaxation times of the medium into which they are introduced. In nuclear imaging, radioactive species are used to generate a signal that is used to visualize the locus within the body at which the radioactive species are located.

Many attempts have been made to develop new formulations containing contrast agents that exhibit improved performance. Such improvements may involve increases in the residence time of contrast agent in the body, and improvements in the specificity with which the agent is delivered, ie concentration of the agent at a particular locus within the body.

In nuclear imaging, radioactive metal ions have been attached directly to a monoclonal antibody (Mab) using a chelating moiety such as diethylenetriamine pentaacetic acid (DTPA). However, this chemistry has been found to be nonspecific and generally leads to a reduction in antibody binding activity, the reduction increasing with increasing amounts of agent coupled to the Mab.

For example, a monoclonal antibody was labelled with DTPA anhydride (DTPAa) and the complex was used to chelate [$^{153}$Sm] chloride (Boniface et al 1989, J Nuc/ Med 30:683-691). When loaded with twenty metal ions, the complex retained antibody binding activity of greater than 90%. However, when the number of metal ions was increased to fifty, the immunoreactivity dropped to below 50%. Clearly, the labelling of antibodies with a metal is limited in terms of the molar ratio that can be bound before antibody binding activity is affected adversely.

Other workers have shown that a DTPA:Mab ratio as low as 2:1 leads to a distinct loss of antibody binding activity (Paik et al 1987, J Nucl Med 24:1158-1163).

The direct labelling of antibodies has been used to produce a number of commercial antibody-based products. For example CEA-scan™ and Onco-scint™ are commercially available agents that can be used to image cancer. These agents consist of antibodies labelled directly with $^{99m}$Tc and on intravenous administration they bind to cancer cells.

A similar approach has been taken in the field of MRI, in which early studies looked at labelling monoclonal antibodies with DTPA-Gd using DTPAa (Unger et al 1985, Investigative Radiol 20(7):693-700). However, the levels of Gd loading were very low (1.5 Gd$^{3+}$ ions per antibody molecule), and no antibody-DTPA-Gd enhancement of the images could be seen in an *in vivo* model. It was postulated that at least 100 Gd atoms would need to be bound to each antibody molecule to produce a signal high enough to detect by MRI.

Other approaches along similar lines have been reported in the literature (for example : Shahbazi-Gahrouei et al 2002, Aust Phys and Eng Sci in Med 25(1):31-38, Matsumura et al (1994), Acta neurochirurgica 60:356-358, Curtet et al (1988), Intl J Cancer 2:126-132).

A common problem with all approaches involving the direct conjugation of a contrast agent with a targeting moiety such as an antibody is that the loading of contrast agent must be maintained at a low level, since otherwise the immunoreactivity of the antibody is adversely affected. The loading of contrast agent may consequently be lower than would otherwise be desired, leading to lower than desired contrast effect. Similarly, where a therapeutic agent is conjugated with a targeting moiety, the efficacy of that agent may be compromised by the need to maintain relatively low loading of the agent.

To increase the residence time of contrast agent in the blood pool, polymeric molecules have been used as a carrier for the contrast agent. In early experiments, carriers such as human albumin and polylysine were labelled with DTPA and used to carry MRI contrast metals (eg DTPA-Gd bound to polylysine, see Gerhard et al (1994), MRM 32: 622-628). In other studies, bovine serum albumin and bovine immunoglobulin were labelled with DTPA and Gd (Lauffer et al (1985), Magnetic Resonance Imaging 3(1):11-16), and a polypeptide based on poly-L-lysine backbone was reacted with DTPAa and used to chelate $^{111}$In for imaging purposes (Pimm et al (1992), Eur J Nucl Med 19:449-452). In these studies, the carrier-bound contrast agent was found to have an increased residence time in the blood pool.

Attempts have been made to combine the two general approaches outlined above, ie to utilise a carrier molecule to increase the loading of contrast agent and to use a targeting moiety to improve specificity.

In one study (Gohr-Rosenthal et al (1993), Invest Radiol 28: 789-795), polylysine labelled with DTPA-Gd was used as an MRI contrast agent. On average, a loading of 65 Gd ions per polymer was achieved. This was coupled to a monoclonal antibody and used to assess human tumours on nude mice. A Mab was linked to the polylysine-DTPA, after activation of the antibody with periodate, by reductive amidation and using a 200-fold molar excess of the polymer. $Gd^{3+}$ was then added at ten-fold molar excess. Antibody binding studies showed the antibody binding activity was reduced by 60-70% compared with the free antibody. It was also noted that the polylysine-antibody conjugate was immunogenic and was taken up by major organs such as the liver and kidney.

Another example of the conjugation of a poly(L-lysine) chain loaded with DTPA-Gd with a Mab was described by Manabe et al (1986), Biochemica at Biophysica Acta 883(3):460-467.

Similar approaches have been taken in relation to the delivery of drugs to the body. For example, the anticancer agent methotrexate (MTX) has been linked to human serum albumin (Pimm et al 1988, in "Human tumour xenografts in anticancer drug development", pp95-98, publ Springer Verlag). Up to 30 MTX molecules were chemically linked to the albumin, and the resulting conjugate was chemically linked to an antibody. However, the antibody-albumin-MTX conjugate had much reduced blood pool half-life in comparison to the free antibody.

Similar work with MTX was done by Hartung et al (1999), Clin Cancer Res 5(4):753-759 in which much lower levels of MTX were loaded onto the HSA molecule. With this reduced drug loading, the half-life of the drug was estimated to be up to 3 weeks.

WO 94/12218 and US4794170 describe the use of lactosaminated human albumin to target antiviral nucleoside conjugates to the liver for the treatment of chronic hepatitis B. WO 90/01900 describes a carrier system for the targeted delivery of MRI contrast agents to specific cell types. This comprises a receptor specific ligand for the cell type chemically bonded to the complexing agent for a paramagnetic material. Poly(L-lysine) is coupled to an asialoglycoprotein targeting agent to increase the number of chelating groups per targeting group. However, the poly(L-lysine) coupling is performed prior to the attachment of the chelating groups and therefore modification of the poly(L-lysine) carrier and the asialoglycoprotein targeting agent will necessarily occur, with a potentially deleterious effect on the binding activity of the complex.

Further approaches to the targeted delivery of agents to the body have involved the use of molecular aggregates (eg liposomes) and particles, typically with dimensions of less than 10$\mu$m.

Examples of work done with liposomes include:

US 5929044 describes delivering a bioactive compound to a cell using a targeting agent. The bioactive agent is carried in either a liposome or a virus.

A large number of publications on the use of liposomes as carriers/targeted carriers of imaging agents are described by Caride (1985) Critical reviews in therapeutic drug carrier systems 1(2) 121-153 and Tilcock (1999) Adv Drug Deliv Reviews 37: 33-51.

DTPA-Gd has been chemically linked to stearylamine and incorporated into the surface of liposomal membranes. These particles had increased relaxivity compared to DTPA-Gd alone (Kunimasa et al (1992) Chem and Pharm Bulletin (Japan) 40(9): 2565-2567).

Another area in which much work has been done is the use of nanoparticles to deliver contrast agents, and the targeting of these nanoparticles to deliver agents to the site of disease.

Examples of work done with nanoparticles include:

Imaging thrombus using fibrin-targeted Gd-DTPA-bis-oleate nanoparticles and similar targeting with Gd-DTPA-phosphatidylethanolamine (PE) nanoparticles using fibrin is described by Winter et al (2003) Magnetic Resonance in Medicine 50(2): 411-416.

Several groups have described the use of targeted iron oxide nanoparticles. For example, Suwa et al (1998) Intl J Cancer 75 (4): 626-634 described nanoparticles of superparamagnetite (T2 contrast agent) coated with Mab directed against epidermal growth factor. Similar work was done by Suzuki et al (1996) Brain Tumour Pathology 13 (2): 127-132 using Mab-labelled polyethylene glycol-coated iron nanoparticles.

In another approach, Artemov et al (2003) Magnetic Resonance in Medicine 49(3): 403-408 described first targeting the tyrosine kinase Her-2/neu receptor with a Mab that had been biotinylated. Then streptavidin labelled superparamagnetite nanoparticles were used to target the biotinylated cancer cells. The nanoparticles generated strong T2 MRI contrast.

A T1 contrast agent has been described by Yu et al (2000) Magnetic Resonance in Medicine 44(6): 867-872 which consists of a fibrin targeted lipid-encapsulated perfluorocarbon nanoparticle with numerous Gd-DTPA complexes incorporated on the outer surface. This was shown to bind strongly to clots.

Another perfluorocarbon based nanoparticle was described by Anderson et al (2000) Magnetic Resonance in Medicine 44(3): 433-439 in which Gd-perfluorocarbon nanoparticles were targeted to alpha(v)beta(3) integrin present on angiogenic endothelium (using a specific Mab).

Mab against human bladder cancer has been linked to adriamycin-loaded human albumin nanospheres by Samten (1996) Chinese J Microbiol and Immunol 16: 54-57.

The use of microspheres as a carrier/targeted carrier has been described in a number of papers. For example, Klibanov (1999) Adv Drug Deliv Reviews 37: 139-157 described gas-filled microbubbles that can be labelled and used as a targeted contrast agent. WO 03/015756 describes a method for the preparation of microspheres of protein material and the incorporation into those microspheres of therapeutic agents and contrast agents.

There have been many publications that describe the delivery of drugs linked to albumin microparticles. For example, cytotoxic drugs (doxorubicin and mitomycin c) have been loaded onto $40\mu m$ human albumin microspheres and administered to the livers of patients with colorectal hepatic metastases (Kerr et a/ (1992) EXS (Switzerland) 61 339-345).

Despite the volume of work that has been carried out, there remains a need for targeted conjugates of agents for delivery to the body that satisfy the conflicting requirements for sufficient loading of the conjugate with the agent concerned and retention of binding activity of the targeting moiety, for effective targeting.

It is therefore an object of the invention to provide means for the targeted delivery to the body of an agent, in particular a contrast agent, by conjugation of a carrier with the agent and with a targeting moiety that directs the conjugate to an intended site of action, which conjugate is able to accommodate a high loading of the agent while maintaining a sufficiently high binding activity of the targeting moiety.

Another object of the invention is to enable the association of large numbers of contrast agents with one or more targeting moieties, without destroying the binding activity of the targeting moieties, as might be caused by the formation of large numbers of covalent bonds with the targeting moieties.

It is a further object of the invention to provide means for the targeted delivery to the body of metal ions in high quantities, while maintaining a high degree of specificity in the localisation of those metal ions within the body.

It is a further object of the invention to provide methods for the assembly of targeted conjugates for the delivery of agents to the body, which methods enable the conjugates to carry high loadings of the agents concerned, while maintaining a high degree of specificity in the targeting of the conjugates.

SUMMARY OF THE INVENTION

According to a first aspect of the invention, there is provided a conjugate for use in medical imaging, which conjugate comprises a carrier in the form of a protein molecule or a particle formed from protein molecules, the carrier being bound to a contrast agent, or a precursor thereof, and to a targeting moiety having an affinity with a specific locus within the body, wherein the carrier molecule is coupled to the targeting moiety by a heterobifunctional cross-linking agent having one reactivity that is specific to functional groups present on the carrier and absent on the targeting moiety, and another reactivity that is specific to functional groups present on the targeting moiety and absent from the carrier.

By a "precursor" of a contrast agent is meant a moiety that is not in itself effective as a contrast agent but which can be rendered so effective by reaction or admixture with some other species prior to use. An example of such a precursor is a metal-chelating moiety, capable of forming bonds with metal ions so as to form a metal chelate that functions as a contrast agent.

By "medical imaging" is meant any technique used to visualise an internal region of the human or animal body, for the purposes of diagnosis, research or therapeutic treatment. Such techniques include principally X-ray imaging, MRI, and nuclear imaging, including PET. Agents useful in enhancing such techniques are those materials that enable visualization of a particular locus, organ or disease site within the body, and/or that lead to some improvement in the quality of the images generated by the imaging techniques, providing improved or easier interpretation of those images. Such agents are referred to herein as contrast agents, the use of which facilitates the differentiation of different parts of the image, by increasing the "contrast" between those different regions of the image. The term "contrast agents" thus encompasses agents that are used to enhance the quality of an image that may nonetheless be generated in the absence of such an agent (as is the case, for instance, in MRI), as well as agents that are prerequisites for the generation of an image (as is the case, for instance, in nuclear imaging).

It will be appreciated that, though the conjugate comprises "a contrast agent" and "a targeting moiety", in practice a substantial number of contrast agents (or precursors thereof) will be bound to each carrier, and more than one targeting moiety may be bound to the carrier. In general, where the carrier has the form of a protein molecule, the number of contrast agents bound to each carrier may be several tens, eg 10 to 100, more preferably 20 to 60, eg 20 to 50. A similar number of contrast agents may be bound to each protein molecule that makes up a carrier in the form of a particle. However, since a particle will generally be formed from a very large number of individual protein molecules (perhaps $10^3$ to $10^{11}$ molecules), the overall number of contrast agents associated with each particle will be correspondingly large. Where the carrier has the form of a protein molecule, the number of targeting moieties per carrier will generally be less than 5, and may just be one. In the case of a particulate carrier, the number of targeting moieties may, and generally will, be considerably greater. Where the carrier is in the form of a protein molecule, it is also possible for more than one carrier to be coupled to a single targeting moiety, though the number of carriers per targeting moiety will generally be less than 10, and is commonly 1, 2 or 3. In general, it is preferred for only a small number of carriers to be bound to the targeting moiety, as excessive numbers of bonds between the targeting moiety and carriers may have an adverse effect on the binding activity of the targeting moiety.

The conjugate according to the invention will generally be administered to the body as a formulation comprising a pharmaceutically acceptable liquid medium. That medium will generally be an aqueous medium, most commonly an aqueous solution containing appropriate excipients. Such excipients may include one or more tonicity-adjusting agents, preservatives, surfactants, and other conventional pharmaceutical excipients.

The conjugate according to the invention may be soluble in the liquid medium, in which case the formulation will generally be a solution of the conjugate. Alternatively, where the carrier is a particle, the particle and hence the conjugate will generally be insoluble, and the formulation will be a suspension or dispersion of the conjugate in the liquid medium.

Thus, according to a second aspect of the invention there is provided a formulation comprising a conjugate according to the first aspect of the invention in admixture with a pharmaceutically acceptable liquid medium.

The conjugate and formulation according to the present invention are advantageous in a number of respects. First, they may prolong the residence time in the bloodstream of the contrast agent. Furthermore, the presence of the targeting moiety, having an affinity with a particular organ or site of disease, enhances delivery of the contrast agent to that location, and may alter the biodistribution of those agents, for example by causing the contrast agent to accumulate in a particular organ or disease site, eg the liver or a tumour, thereby allowing that organ or disease site to be targeted and visualised. The use of a "carrier" for the contrast agent may increase the quantity of contrast agent delivered to the desired site within the body. This may enhance detection due to an increase in signal/noise ratio against background (non-diseased) tissue. The use of the targeting moiety avoids delivery of agent to normal/healthy tissue. In addition, as explained below, large quantities of contrast agent may be conjugated to the carrier without damaging the binding capability of the targeting moiety.

MRI contrast agents that may be employed in the invention include metal ions, notably gadolinium. Such ions may be coupled to the carrier material via a chelating moiety that is covalently bound to the carrier material.

Similarly, metals useful in nuclear imaging, eg $^{99m}Tc$, $^{201}Tl$ and $^{111}In$, may also be coupled to the carrier material, either directly or indirectly, eg via a chelating moiety.

In a similar manner to the way in which metals effective as contrast agents may be delivered to the body in the form of a conjugate according to the first aspect of the invention, other metals may also be delivered to the body for other purposes. Some metals, for instance, may have a direct therapeutic effect, eg radioactive metals useful in radiotherapy. One such radioactive metal is $^{67}Cu$, which may be bound to the carrier in an analogous manner to the metals used in imaging techniques. The resulting conjugate can be used for targeted radiotherapy.

Thus, in another aspect of the invention, there is provided a conjugate for the delivery of a metal to the body, which conjugate comprises a carrier in the form of a protein molecule or a particle formed from protein molecules, the carrier being coupled via a chelating agent to said metal and conjugated with a targeting moiety having an affinity with a specific locus within the body, wherein the carrier molecule is coupled to the targeting moiety by a heterobifunctional cross-linking agent having one reactivity that is specific to functional groups present on the carrier and absent on the targeting moiety, and another reactivity that is specific to functional groups present on the targeting moiety and absent from the carrier.

As described above, the metal may be a metal that is useful as a contrast agent, eg in MRI or nuclear imaging, or it may be a metal useful in therapy, eg a radioactive metal useful in radiotherapy. It is also possible to prepare conjugates containing more than one type of metal, eg a mixture of a contrast agent (eg gadolinium) and a radio-therapeutic agent (eg $^{67}Cu$). It is also possible to prepare formulations comprising more than one conjugate, eg a first conjugate comprising a contrast agent and a second conjugate comprising a radio-therapeutic agent. By such means, it is possible to determine the precise delivery and location of the agent in the body using conventional imaging techniques and in so doing to confirm successful delivery of the radioisotope.

It may also be possible to prepare particles of larger size (eg over $10\mu m$) that may carry radioisotopes and/or imaging agents. These can be delivered (eg by catheter) into the microcirculation of a tumour and so reduce blood supply by capillary blockade. In addition, where a radioisotope is present on the particle, it will be delivered into the tumour (resulting in cell death), and the presence of a contrast agent, eg gadolinium, will enable the tumour to be imaged over a period of time using conventional imaging technology (eg MRI).

The conjugate according to the first aspect of the invention may be prepared by reacting the carrier with the contrast agent, or with a precursor thereof, in the absence of the targeting moiety, and subsequently coupling the carrier to the targeting moiety using a heterobifunctional cross-linking agent. This approach is more generally applicable, being useful in the preparation of targeted conjugates of a variety of carriers with agents for delivery to the body. Thus, according to a further aspect of the invention, there is provided a method for the preparation of a targeted conjugate of an agent for delivery to the body with a carrier, which method comprises

(a) reacting the agent for delivery to the body, or a precursor thereof, with the carrier to form an intermediate conjugate, and thereafter
(b) (i) reacting the intermediate conjugate with a heterobifunctional cross-linking agent to activate the intermediate conjugate and then reacting the activated intermediate conjugate with a targeting moiety, or (ii) reacting the intermediate conjugate with a targeting moiety that has been activated by reaction with a heterobifunctional cross-linking agent, or (iii) causing the intermediate conjugate, a heterobifunctional cross-linking agent and a targeting moiety to react simultaneously together,

wherein the cross-linking agent has one functionality that is specific for reaction with groups present on the carrier and absent from the targeting moiety, and a second functionality that is specific for reaction with groups present on the targeting moiety and absent from the carrier.

It is particularly preferred that reaction of the carrier with the agent for delivery to the body (or precursor thereof) and with the heterobifunctional cross-linking agent should be via two different types of functional group present on the carrier, and that reaction of the heterobifunctional cross-linking agent with the targeting moiety should take place via a third type of functional group present on the targeting moiety, the three types of functional group being different to each other.

The method according to this aspect of the invention is advantageous in that reaction of the carrier with the agent for delivery to the body (or precursor thereof) and reaction of the carrier with the targeting moiety are carried out in separate steps. The carrier is thus reacted with the agent for delivery to the body before it is conjugated with the targeting moiety, and reaction of the agent for delivery to the body with the targeting moiety is avoided. A high loading of agent on the carrier, and hence on the final conjugate, can thereby be achieved with minimal, or no, adverse effect on the binding activity of the targeting moiety. Conjugates prepared in accordance with the method may therefore be characterised by the absence of agent for delivery to the body (or precursor thereof) from the targeting moiety. To put this another way: the conjugate is preferably such that at least 90%, more preferably at least 95%, and most preferably at least 99% of the agents for delivery to the body (or precursors thereof) are covalently bound to the carrier, rather than to the targeting moiety. This leads to minimal loss of binding activity, and the conjugate prepared in accordance with the invention may therefore be characterised in that the binding activity of the targeting moiety in the conjugate, as measured in a competitive binding assay, is at least 50%, more preferably at least 60%, 70%, 80% or at least 90%, that of the free targeting moiety.

Where the carrier has the form of a particle, the method according to the invention may include the preliminary step of forming the particle from particle-forming material, the particle constituting the carrier that is then reacted with the agent for delivery to the body (or precursor thereof) and subsequently with the targeting moiety.

In a variation on this method, the step of forming the particle may take place after the agent for delivery to the body, or precursor thereof, has been reacted with the particle-forming material.

Where the carrier is reacted with a precursor of the agent that is to be delivered to the body, the precursor will subsequently be converted to that agent. Such conversion may take place before, after or simultaneously with step (b) of the method. Where the precursor is a chelating agent and the conversion involves formation of a chelate between the chelating agent and metal ions, it is preferred that the pH of the reaction mixture is maintained, during addition of the metal ions, between 5.0 and 6.5.

The method is particularly applicable to the preparation of conjugates in which the carrier is proteinaceous.

The cross-linking agent has one functionality that is specific for reaction with groups present on the carrier and absent from the targeting moiety, eg sulphydryl groups, and a second functionality that is specific for reaction with groups present on the targeting moiety and absent from the carrier, eg aldehyde groups. This reduces or eliminates the occurrence of unwanted side reactions that would lead to the coupling together of carriers and/or targeting moieties. The method is also particularly applicable to the preparation of conjugates in which the agent for delivery to the body has, or is coupled to the carrier via an intermediate compound or moiety that contains, carboxyl groups or derivatives thereof, in which case the coupling with the carrier may be by means of amide bonds formed between those carboxyl groups and amino groups present in the carrier.

Where the heterobifunctional cross-linking agent reacts with sulphydryl groups present on the carrier, it has been found that the free sulphydryl groups on the intermediate conjugate are reversibly blocked during step (a) of the method. In such a case, the method preferably includes an intermediate step (between steps

(a) and (b)) of unblocking free sulphydryl groups. Such unblocking is preferably carried out by incubation of the intermediate conjugate, eg at a temperature of between 20˚ and 50˚C, for a period of between 1 and 24 hours.

According to a yet further aspect of the invention, there is provided the use of a conjugate according to the first aspect of the invention in the manufacture of a formulation for enhancing the contrast of an image to be obtained by a medical imaging technique.

DETAILED DESCRIPTION OF THE INVENTION

Nature of the carrier material

The conjugates according to the first aspect of the invention comprise protein as a carrier material for the contrast agent and the targeting moiety.

Proteins that may be used as carrier materials include globular proteins and fibrous or structural proteins, and mixtures thereof.

Examples of globular proteins include synthetic or natural serum proteins, natural or synthetic derivatives thereof, salts, enzymatically, chemically, or otherwise modified, cleaved, shortened or cross-linked, oxidised or hydrolysed derivatives or subunits thereof. Examples of fibrous or structural proteins include synthetic or natural collagen, elastin, keratin, fibroin, fibrin, and fibronectin, natural or synthetic derivatives thereof, and mixtures thereof. Examples of serum proteins are albumin, $\alpha$-globulins, $\beta$-globulins, $\gamma$-globulins, transthyretin, fibrinogen, thrombin and transferrin.

The protein is most preferably a single, complete or substantially complete, protein molecule. However, the protein molecule may be an oligomer of conjugated complete or substantially complete protein molecules. Such an oligomer may be a protein dimer, or trimer, or higher oligomer comprising up to, say, twenty discrete protein molecules, more preferably up to ten, or up to five, discrete protein molecules.

The protein molecule may alternatively be a fragment of a complete protein molecule, by which is meant a molecule comprising a sequence of amino acids that corresponds to a sequence of amino acids found in a naturally-occurring protein molecule, but which is shorter in length. Such a fragment, however, preferably comprises a sequence of amino acids that has a length of more than 50%, 60%, 70%, 80%, or 90% and most preferably more than 95% that of a naturally-occurring protein molecule, and which has a degree of homology of greater than 80%, 90% or most preferably greater than 95% with the corresponding part of the naturally-occurring protein molecule.

Transferrin may have particular benefits as a carrier material in that it has numerous potential coupling sites, it may facilitate transport of a conjugate according to the invention across the blood-brain barrier, and it may be prepared as a recombinant product (see, for example, MacGillivray et al 2002, in Molecular and Cellular Iron Transport, Templeton (Ed), Marcel Dekker, Inc._p 41 and Mason et al 1993. Biochemistry 32: 5472).

The particularly preferred carrier material is albumin, for the reasons detailed below.

Where the conjugates are intended for administration to the human body, the carrier material is preferably of human origin, ie actually derived from humans, or is identical (or substantially so) in structure to protein of human origin. A particularly preferred carrier material is thus human serum albumin.

Human serum albumin may be serum-derived, for instance obtained from donated blood. However, in order to eliminate or reduce the risk of transmission of potential contaminants, eg viral or other harmful agents, that may be present in blood-derived products, as well the potential limitations on supply associated with material isolated from donated blood, it is preferred that the carrier material, eg human serum albumin, should be a recombinant product derived from microorganisms (including cell lines), transgenic plants or animals that have been transformed or transfected to express the carrier material.

The presently most-preferred carrier material for use in the present invention is thus recombinant human serum albumin (rHA). Suitable forms of rHA may be obtained commercially from Delta Biotechnology Ltd, Nottingham, United Kingdom.

Processes for the preparation of rHA will in general be familiar to those skilled in the art and are described, for instance, in WO 96/37515 and WO 00/44772.

In a preferred process for the preparation of rHA, an initial rHA solution is derived from a fungal culture medium obtained by culturing a fungus transformed with an rHA-encoding nucleotide sequence in a fermentation medium, whereby said fungus expresses rHA and secretes it into the medium. The fungus may be a filamentous fungus such as an *Aspergillus* species. Preferably, the fungus is a yeast. More preferably, the fungus is of the genus Saccharomyces (eg *S. cerevisiae*), the genus *Kluyveromyces* (eg *K. lactis*) or the genus *Pichia* (eg *P. pastoris*).

The rHA preferably contains a substantial proportion of molecules with a free -SH (sulphydryl or thiol) group. This provides a particularly useful means of conjugation of the rHA molecule to a targeting moiety, as described below.

Where the conjugation chemistry according to the invention is applied to a non-protein carrier, any one of a variety of carrier materials may be employed. The carrier material should be biocompatible and should be such that the conjugates of the carrier material with the targeting moiety maintain their integrity prior to use and for the duration of their useful life *in vivo*. It is strongly preferred that the carrier material should have two different types of functional groups, enabling different chemical methods to be used for coupling of the carrier material to the agent for delivery to the body (or precursor thereof) and to the targeting moiety.

Other, non-proteinaceous, materials that may be used as carrier materials include polysaccharides, as well as suitable synthetic polymers.

The carrier material is, however, most preferably proteinaceous. Where the carrier is in particulate form, the carrier material (or particle-forming material) may be insoluble or the particle may formed from a soluble material and then rendered insoluble by subsequent treatment, eg thermally-induced cross-linking. For example, the carrier material may be collagen or gelatin.

Where the carrier is not in particulate form, then the carrier material is preferably soluble.

Albumin is the currently most preferred carrier material for both soluble and particulate conjugates, for the following reasons:

a) albumin is soluble in aqueous media;
b) particles of albumin may readily be rendered insoluble;
c) the free sulphydryl group present in the albumin molecule provides a means for selective coupling to the targeting moiety; and
d) albumin contains numerous amino acid residues with pendant amino groups (specifically lysine residues) that provide coupling sites for agents for delivery to the body.

Formation of particles

Particles of particle-forming material may be produced by any suitable technique, and numerous such techniques will be familiar to those skilled in the art.

A particularly preferred method for making the particles according to the invention comprises the steps of

i) forming a suspension of the particle-forming material; and
ii) spray-drying said suspension.

Step i), ie the formation of a suspension of the particle-forming material, is preferably carried out by first dissolving the particle-forming material in a solvent, and then adding to the solution so formed a non-solvent for the particle-forming material, so as to bring about precipitation of the particle-forming material. By a "non-solvent" is meant a liquid in which the solubility of the particle-forming material is substantially less than the solubility of the particle-forming material in the solvent, but which is miscible with the solvent.

The non-solvent is preferably added in excess, ie the volume of non-solvent added to the solvent is preferably greater than the volume of the solution of the particle-forming material in the solvent. In other words, the solvent / non-solvent mixture that is spray-dried in step ii) most preferably comprises in excess of 50% v/v of non-solvent, more preferably in excess of 60% v/v, and possibly in excess of 70% v/v.

Most commonly, the solvent is water. The preferred non-solvent is ethanol. In general, however, any suitable combination of solvent and non-solvent may be used, provided that the addition of non-solvent has the desired effect of causing precipitation of the particle-forming material and that the solvent and the non-solvent are miscible in the proportions used.

Although the solvent is most commonly water, it may alternatively be, for example, an organic solvent. In such a case, the non-solvent may be water, and the use of the non-solvent may then be beneficial in reducing risks associated with the subsequent spray-drying of the suspension containing the possibly flammable organic solvent.

Where, as is most commonly the case, the particle-forming material is a proteinaceous material, the precipitation by addition of the non-solvent is preferably carried out at a pH which is removed from the isoelectric point, so as to prevent or minimise agglomeration of the suspended particles and to produce hydrophilic particles that are readily susceptible to dispersion after spray-drying. In this way, the use of additional surfactants to achieve the same objectives may be avoided.

Step ii), ie spray-drying of the suspension formed in step i), may be carried out in a generally conventional manner, using equipment of a generally conventional nature. In outline, the spray-drying process involves spraying the suspension into a chamber containing a heated gas, most commonly air. This causes the solvent/non-solvent mixture to evaporate and produces solid particles. The gas is drawn from the chamber, and the particles entrained in the gas are separated from the gas, eg by means of a cyclonic separator or some form of filter arrangement. The particles are then collected in a suitable receptacle.

The properties of the particles obtained by the spray-drying process are dependent on a number of factors. These include the flow rate of gas through the spray-drying apparatus, the concentration of the particle-forming material in the suspension, the nature of the solvent and non-solvent, the rate at which the suspension is fed into the spray-drying apparatus and the temperature of the gas in the chamber. Usually, small size distributions can be achieved by a combination of a low suspension feed rate, appropriate nozzle design, a high degree of atomization and high flow rate of gas.

It is particularly preferred that, between step i) and step ii), ie after formation of the suspension but before spray-drying, the suspension is subjected to homogenisation, eg by mechanical agitation. This results in a more even size distribution of particles in the suspension, and a correspondingly smaller and more even size distribution of the particles formed in step ii).

The suspension preferably contains from 0.1 to 50% w/v of particle-forming material, more preferably 1 to 20% w/v, and most preferably 2 to 10% w/v, particularly when the particle-forming material is albumin. Mixtures of particle-forming materials may be used, in which case the above figures represent the total content of particle-forming material(s).

After spray-drying of the particles, it may be necessary or desirable for the particles to be rendered insoluble. This may be achieved by cross-linking of the particle-forming material, which may be brought about by a variety of techniques that are known _per se_. In a preferred method, the particles are rendered insoluble by heat treatment, eg at a temperature in excess of 150°C for a period in excess of 30min, eg 1 hour or several hours.

The preferred method for preparing particles as described above is advantageous in that the two stages of the process (formation of a suspension and spray-drying) may be optimised separately to achieve the desired form and size distribution of particle. This gives a high degree of control over the properties of the particles. In particular, the process enables the production of particles with particularly small sizes and particularly narrow size distributions. Particles produced by this method may, for example, have particle sizes of predominantly less than $4\mu m$, and number sizes with modal peaks below $1 \mu m$ and mean sizes (as measured using a Coulter counter) less than $2\mu m$. Particles with such small sizes are beneficial in that they may enter very small blood vessels (capillaries) and/or may penetrate deep into the lungs. It may also be possible to produce particles of larger size, eg for nasal administration.

It will be appreciated that references to the "size" of the particles will normally mean the "diameter" of the particles, since the particles will most commonly be substantially spherical. However, it will also be appreciated that the particles may not be spherical, in which case the size may be interpreted as the diameter of a notional spherical particle having a mass equal to that of the non-spherical particle.

Coupling of agents to the carrier material

Coupling of the agent for delivery to the body to the carrier material may be carried out by any of a number of means, depending _inter alia_ on the nature of the agent and the nature of the carrier material. In general, however, coupling will involve the formation of covalent bonds between the carrier material and the agent, or between the carrier material and a coupling moiety capable of forming a chemical or physical bond with the agent itself.

One preferred method of coupling, particularly appropriate to the coupling of metals, eg metals for use in MRI or nuclear imaging, or the coupling of radioactive metals for use in radiotherapy, involves the conjugation of the carrier material with a chelating agent which is capable of binding the metal.

In one particularly preferred embodiment, the chelating agent comprises carboxyl groups, or derivatives thereof, that react with amine groups present in the carrier material (eg proteinaceous carrier material such as albumin) to form amide bonds linking the chelating agent to the carrier material. A solution of a suitable salt of the metal may then be added, leading to chelation of the metal by the conjugated chelating agent.

Chelating agents that may be used include acetic acid derivatives of compounds containing multiple amine groups. Examples include ethylenediamine tetraacetic acid, diethylenetriamine pentaacetic acid, and derivatives thereof, eg diethylenetriamine pentaacetic acid anhydride. Other classes of chelating agent that may be useful include macrocyclic chelating agents. Examples of macrocyclic chelators are:

1,4,7-triazacyclononane-N,N',N''-triacetic acid (NOTA)
1,4,7,10-tetraazacyclododecane-N,N',N'',N'''-tetraacetic acid (DOTA)
1,4,8,11-tetraazacyclotetradecane-N,N',N'',N'''-tetraacetic acid (TETA)

Other methods for coupling chelating agents to carrier material will be evident to those skilled in the art. Suitable chemistries most commonly involve the formation of linkages through amine, thiol, carbonyl, carboxyl or hydroxyl groups present in the carrier material and/or the chelating agent.

Where the agent is coupled to the carrier in the form of a metal chelate, the chelate may be formed as part of the manufacturing process, or alternatively the metal may be added later, eg just prior to use. Particularly where the metal is a radioactive metal, it may be desirable for the metal ions to be added to the formulation immediately prior to use.

Likewise, organic agents, such as the iodine-containing compounds referred to below that are used as X-ray contrast agents may be coupled directly to the carrier material by the formation of covalent bonds between the organic agent and the carrier material. Methods for coupling organic agents to carrier material will again be evident to those skilled in the art, and may involve the formation of linkages through amine, thiol, carbonyl, carboxyl or hydroxyl groups present in the carrier material and/or the organic agent.

In general, more than one molecule of organic agent or chelating agent is coupled to the carrier, more preferably in excess of ten such molecules or in excess of twenty such molecules. In the preferred case of a proteinaceous carrier material, eg albumin, it may be possible to couple between 10 and 100, more preferably between 20 and 60, molecules of organic agent or chelating agent to each protein molecule, eg 20-50 molecules of such agent per protein molecule. This leads to a considerably increased density of such agents, compared with conventional delivery systems for MRI contrast agents or radioactive metals for use in radiotherapy. In many instances, it may be desirable for the loading of organic agent or chelating agent on the carrier to be as high as possible. In other cases, it may be beneficial to restrict the loading to a lower level.

Nature of the targeting moiety

The carrier material is conjugated with a targeting moiety having an affinity with a particular organ or disease site within the body. Such targeting moieties include antibodies, other proteins and peptides. Preferred targeting moieties are antibodies, particularly monoclonal antibodies. Many suitable targeting moieties (eg antibodies) are available commercially.

Examples of antibodies that may be useful as targeting moieties in the present invention include:

| Tumour type | Target antigen | Antibody |
|---|---|---|
| Colorectal cancer | CEA | hMN-14 |
| Breast Cancer | MUC1 | HuBrE3 |
| Bladder Cancer | MUC1 | C595 |
| Prostate Cancer | PSMA | J591 |
| Renal cell carcinoma | Glycoprotein | chG250 |

Further examples of antibodies that may be conjugated with the carrier material include the following:

a) Vitaxin™ - a humanised antibody that binds alpha-v beta-3 expressed on newly formed blood vessels in tumours;
b) humanised CD22 Antibody (normally labelled with Yttrium-90 for treatment of non-Hodgkins lymphoma); and
c) αCD45 antibody to the tyrosine phosphatase CD45 that is expressed on all hematopoietic cells and particularly on lymphocytes.

Examples of other forms of targeting moiety include:

a) fibrin for imaging clots;
b) binding moieties that bind to fibrin;
c) lipophilic and amphiphilic organic molecules;
d) receptor ligands;
e) steroids;
f) lipids;
g) hormones;
h) a synthetic peptide (commercially available from Diatide) that binds to somatostatin receptor on pulmonary cancers;
i) antibody fragments raised against white blood cell antigen - for detecting infectious diseases; and
j) Annexin V, which binds phosphatidyl serine released on cell death - a marker for imaging heart disease and assessing response to chemotherapy.

The effect of the targeting moiety is to concentrate the conjugates, loaded with contrast agent or therapeutic agent, at a desired locus within the body, eg a particular organ or a disease site such as a tumour.

Coupling of carrier to targeting moiety

Methods for coupling targeting moieties to the carrier material are known *per se* and will be familiar to those skilled in the art. Suitable chemistries most commonly involve the formation of linkages through amine, thiol, carbonyl, carboxyl or hydroxyl groups present in the carrier material and/or the targeting moiety.

The carrier is coupled to the targeting moiety using a heterobifunctional cross-linking agent. The cross-linking agent has one reactivity that is specific to functional groups present on the carrier and absent from the targeting moiety, and another reactivity that is specific to functional groups present on the targeting moiety and absent from the carrier. As previously described, this eliminates the occurrence of undesired side reactions such as coupling together of carrier molecules or particles, reaction of both functionalities of the cross-linking agent with the carrier or the targeting moiety, etc.

The cross-linking agent may first be reacted with the intermediate conjugate (ie the carrier after reaction with the agent for delivery to the body, or precursor thereof), thereby activating the intermediate conjugate, and then adding the targeting moiety (eg an antibody) which will react with the other end of the heterobifunctional cross-linking agent. Alternatively, the cross-linking agent may first be reacted with the targeting moiety, and the activated targeting moiety then reacted with the intermediate conjugate. It may also be possible for the intermediate conjugate, targeting moiety and cross-linking agent to be reacted together in a single step. Suitable heterobifunctional cross-linking agents are commercially available.

Preferred cross-linkers for reaction via an -SH (sulphydryl or thiol) group on the carrier have groups that react specifically with sulphydryl groups. One preferred example of such a group is a maleimide group. Other examples are 2-pyridyldithio, haloacetate or haloacetamide, in particular the iodo derivatives, aziridine, acryloyl/vinyl, 4-pyridyldithio and 2-nitrobenzoate-5-dithio.

In the case of a carrier material that does not contain free thiol groups, such groups can be generated on the carrier, eg by reduction with dithiothreitol, or introduced using thiolating agents such as iminothiolane or N-succinimidyl S-acetylthioacetate (SATA).

A preferred method of reacting the cross-linker with the targeting moiety, especially where the latter is an antibody or other protein, is via a carbohydrate moiety on the targeting moeity. Under mild oxidising conditions, cis-diols found in carbohydrates can be converted to aldehyde groups, with which hydrazide groups are specifically reactive.

Particularly preferred heterobifunctional cross-linking agents for use in the invention thus include both an SH-reactive functionality, eg maleimide or 2-pyridyldithio, and an aldehyde-reactive functionality, eg hydrazide.

Preferred heterobifunctional cross-linking agents for use in the invention are thus:

maleimidocaproic acid hydrazide (commonly referred to as EMCH) 3-(2-pyridyldithio)-propionyl hydrazide (PDPH)
maleimidopropionic acid hydrazide (MPH)
N-($\kappa$-maleimidoundecanoic acid) hydrazide (KMUH)
4-(4-N-maleimidophenyl)butyric acid hydrazide (MPBH)

Cross-linking agents including hydrazide groups may be utilised in the form of their acid addition salts, especially the hydrochloride.

As described above, in the preferred case where the carrier material is rHA, a particularly useful method of coupling the targeting moiety to the rHA molecule is by linkage through the free sulphydryl (thiol) group that is present on rHA. As there is no more than one such free sulphydryl group on each rHA molecule, one rHA molecule will couple to only one targeting moiety.

The rHA preferably has a free thiol content of at least 0.85, 0.8, 0.75, 0.7, 0.65 or 0.60 mole SH/mole protein when measured by using Ellman's Reagent, 5,5'-dithiobis-(2-nitrobenzoate) (DTNB), which is a specific means of detecting free sulfhydryl groups such as cys-SH (Cys-residue 34 in the case of rHA). The reaction releases the 5-thio-2-nitrobenzoate ion $TNB^{2-}$ which has an absorption maximum at 412nm. By measuring the increase in absorbance at 412nm and dividing by the molar extinction coefficient of the $TNB^{2-}$ ion at 412nm, the free sulfhydryl content of rHA can be calculated.

Preferably, an antibody targeting moiety will be coupled to the carrier material via a coupling site in the constant region of the antibody, so as to preserve the specific binding activity of the variable region of the antibody.

Nature of contrast agents

The conjugates and formulations according to the invention are useful for the delivery of contrast agents. Imaging techniques in which contrast agents are used include MRI, X-ray imaging techniques and nuclear imaging, including PET.

X-ray contrast agents that may be used in the invention include a variety of iodine-containing compounds that have suitable properties for such use. Such compounds are generally soluble and may be ionic or non-ionic. One particular example of such an X-ray contrast agent is that known as iopamidol. Other known X-ray contrast agents include iomeprol, iopromide, ioversol, iodixanol and iohexol.

MRI contrast agents that may be used include a variety of compounds comprising paramagnetic metal ions. Suitable such ions include manganese and, particularly, gadolinium.

Metals are also used in nuclear imaging. Metals suitable for this application are generally radioactive $\gamma$-emitters. Examples include [99mTc], [201Tl] and [111In].

In addition to metals, it may also be possible to couple to the carrier material non-metallic atoms that are useful in imaging (or to couple compounds containing such atoms or into which such atoms can be introduced). Examples of such atoms include $^{123}$I and $^{121}$I.

Metals useful in radiotherapy

The conjugates and formulations according to the invention may also be used to deliver radioactive metals useful in radiotherapy. Such metals are generally emitters of β-particles, and examples include $^{67}$Cu, $^{153}$Sm, $^{90}$Y, $^{191}$Pt, $^{193}$Pt and $^{195}$Pt.

Administration of the formulations

The conjugates and formulations according to the invention may be administered by a variety of routes. The formulations may, for instance, be administered intravenously. The formulations may also be administered by oral or nasal inhalation, eg as a nebulised solution. Where appropriate, the formulations may be delivered direct to a disease site via a catheter.

EXEMPLARY EMBODIMENTS OF THE INVENTION

The invention will now be described in greater detail, by way of illustration only, with reference to the following Examples and the accompanying drawings. Examples 1 to 10 relate to conjugates based on soluble rHA, and Examples 11 to 19 relate to conjugates based on insoluble rHA particles.

BRIEF DESCRIPTION OF THE DRAWINGS

Figure 1 shows *in vitro* MRI properties of rHA labelled with gadolinium-diethylene triamine pentaacetic acid (Gd-DTPA);

Figure 2 shows the binding of copper ions to DTPA-labelled albumin;

Figure 3 shows the Gd$^{3+}$-binding capacity of an rHA-DTPA conjugate as a function of the amount of DTPAa used in the reaction;

Figure 4 illustrates the feasibility of the separation of rHA-DTPA from excess DTPA by different chromatographic methods;

Figure 5 illustrates the recovery of free thiol in samples of rHA-DTPA and rHA-DTPA-Gd;

Figure 6 shows the extent of reaction of an antibody with PDPH, as indicated by measurement of released 2-thiopyridine;

Figure 7 is a similar plot to Figure 6, for a different antibody;

Figure 8 demonstrates retention of binding activity for a conjugate according to the invention;

Figure 9 shows *in vitro* MRI properties of rHA particles labelled with Gd-DTPA;

Figure 10 shows the particle size distribution of rHA particles before (Figure 10a) and after (Figure 10b) labelling with Gd-DTPA; and

Figure 11 is a photomicrograph of rHA particles labelled with Gd-DTPA after resuspension in water.

Abbreviations

αCD45      mouse anti-rat CD45
C595       monoclonal antibody specific to MUC1 antigen
DTNB       Ellman's Reagent (5,5'-dithiobis(2-nitrobenzoic acid))
DTPA       diethylenetriaminepentaacetate
DTPAa      diethylenetriaminepentaacetic acid anhydride
DTT        dithiothreitol
EMCH       N-(ε-maleimidocaproic acid) hydrazide
GPHPLC     gel permeation high performance liquid chromatography
IgG        Immunoglobulin G
Mab        monoclonal antibody
MRI        magnetic resonance imaging
PBS        phosphate buffered saline (0.9%(w/v) NaCl, 15mM $Na_2HPO_4$, 5mM $NaH_2PO_4$)
PDPH       3-(2-pyridyldithio)-propionyl hydrazide

rHA     recombinant human albumin

XO      xylenol orange

General Methods for Examples 1 to 10

Free thiol assay

Free thiol concentration was determined by reaction with DTNB at pH8 and absorbance measurement at 412nm, using an extinction coefficient of $13600M^{-1}.cm^{-1}$ for the released 5-thio-2-nitrobenzoate.

Protein assay

rHA concentration was determined by absorbance measurement at the peak near 280nm, using a $1g.L^{-1}$ extinction coefficient of 0.53. IgG concentrations were determined similarly, but using a $1g.L^{-1}$ extinction coefficient of 1.43.

GPHPLC

GPHPLC was performed using a TSKgel G3000SWXL 0.78×30cm column and guard (Tosoh Biosep), eluted at $1mL.min^{-1}$ in PBS.

Materials

DTPAa and gadolinium chloride were obtained from Sigma. Recombumin®-25 (25%(w/v) rHA) was obtained from Delta Biotechnology Ltd, Nottingham, UK. Magnevist™ (a 0.5M Gd-DTPA chelate) was available from commercial sources, and was used as a control.

Example 1

Preparation of soluble rHA labelled with MRI contrast agent (gadolinium chelate)

This method describes the development of an MRI contrast agent, based on soluble rHA labelled with DTPA followed by gadolinium (Gd).

1.1 Preparation of rHA labelled with MRI contrast agent (rHA-DTPA-Gd)

rHA was diluted to $20g.L^{-1}$ rHA in water and DTPAa (1g per 0.3g rHA) was added slowly with constant stirring over a period of approximately 30min. During this time, 5M NaOH was added to maintain the pH as close to 8.0 as possible. Stirring was continued for 30min after the final DTPAa addition and the pH then adjusted to 7.0 with 5M HCl. The soluble rHA-DTPA was dialysed overnight against approximately 120vol water to remove excess free DTPA.

Gd labelling was performed by titration with 0.1 M $GdCl_3$. Care was taken to avoid adding excess $GdCl_3$, which resulted in complex formation and precipitation of the albumin. The point of precipitation was thought to be a measure of available DTPA. The resulting bound DTPA level, determined from the point of Gd-induced precipitation, was $46mol.mol^{-1}$ rHA.

1.2 Imaging properties of rHA labelled with MRI contrast agent (Gd-DTPA)

Soluble rHA-DTPA-Gd was prepared essentially as described above, but with the following modifications:

a) the rHA concentration for DTPA labelling was 25g.L$^{-1}$;
b) the DTPAa addition time was $\approx$25 minutes;
c) dialysis was performed against approximately 350vol water for 18 hours followed by approximately 100vol for 2 hours;
d) the resulting bound DTPA level, determined from the point of Gd-induced precipitation, was 45 mol.mol$^{-1}$ rHA.

The resulting material was 0.2$\mu$m filtered, concentrated to approximately 90g.L$^{-1}$ rHA (based on starting rHA) by ultrafiltration (Vivaspin20 10000MWCO centrifugal concentrators at 3300rpm in Sorvall RT6000B centrifuge) and formulated to 50g.L$^{-1}$ rHA in 5%(w/v) mannitol. The resulting Gd concentration was calculated assuming no loss during ultrafiltration. The formulated material was frozen in 2mL aliquots with gentle agitation in a -30˚C bath and stored frozen (approximately -20˚C) until required.

Magnetic resonance imaging was performed with the soluble rHA-DTPA-Gd and the Magnevist™ control diluted appropriately to 2mL with water, then mixed with 8mL 0.5% agarose to give the specified Gd concentration. Relaxation rates (R1 and R2) were determined for each sample, using a range of repetition times (TR) and echo times (TE), by fitting the data to either

$$M = M_\infty(1 - e^{-R1.TR}) \quad \text{or} \quad M = M_0(e^{-R2.TE})$$

where M is the measured signal at time T
$M_0$ is the signal at T=0
and $M_\infty$ is the signal at T=$\infty$.

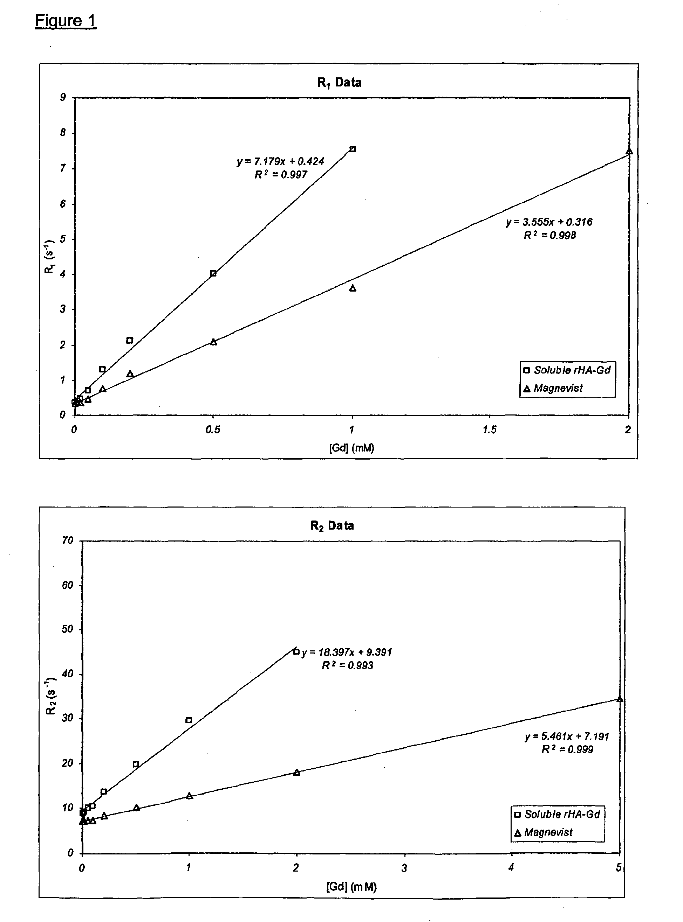

The *in vitro* MRI properties of this soluble rHA-DTPA-Gd were compared with those of Magnevist™, a commercial MRI contrast agent based on Gd-DTPA (Figure 1). Both R1 and R2 relaxation rates were significantly greater than those obtained with Magnevist™. This indicated that soluble rHA-DTPA-Gd should produce *in vivo* images equivalent to or better than those seen with Magnevist™ at the same Gd concentration, particularly in view of the marked reduction in clearance rate anticipated for the rHA based material. Alternatively, it suggested that soluble rHA-DTPA-Gd could be used at a lower Gd dose than Magnevist™.

Example 2

Binding of copper ions to soluble rHA labelled with DTPA

The recombinant human albumin labelled with DTPA (as prepared in Example 1) can be used as a carrier for radioactive metals such as copper, indium, technetium and others.

To confirm this, DTPA-labelled albumin (prepared as in Example 1 above) can be shown to bind copper ions (for this Example non-radioactive copper was used).

2.1 Preparation of rHA-DTPA

A sample of rHA-DTPA was prepared as described above in Example 1.3, but with the following modifications:

a) the DTPAa addition time was $\approx$20min;
b) the stirring time post DTPAa addition was $\approx$20min;
c) dialysis was performed against approximately 350vol water for 4h, followed by approximately 350vol for 16h and approximately 100vol for 3h;
d) the preparation was halted prior to GdCl$_3$ titration;
e) the resulting bound DTPA level, determined from the point of Gd-induced precipitation with a small sample of the rHA-DTPA, was 46mol.mol$^{-1}$ rHA.

2.2 Spectrophotometric $Cu^{2+}$ titration

A 5mL aliquot of rHA-DTPA was adjusted to pH6 with 0.5M NaOH and the total volume recorded. A sample was taken for $A_{700}$ measurement and then returned to the bulk. Twelve 50$\mu$L aliquots of 0.1 M $CuSO_4$ were added with pH adjustment and $A_{700}$ measurement, as above, following each addition. Measured absorbance values were corrected back to a volume of 5mL and plotted against the amount of Cu added. Regression lines were fitted to the two phases of the titration and their intersection taken as the end point.

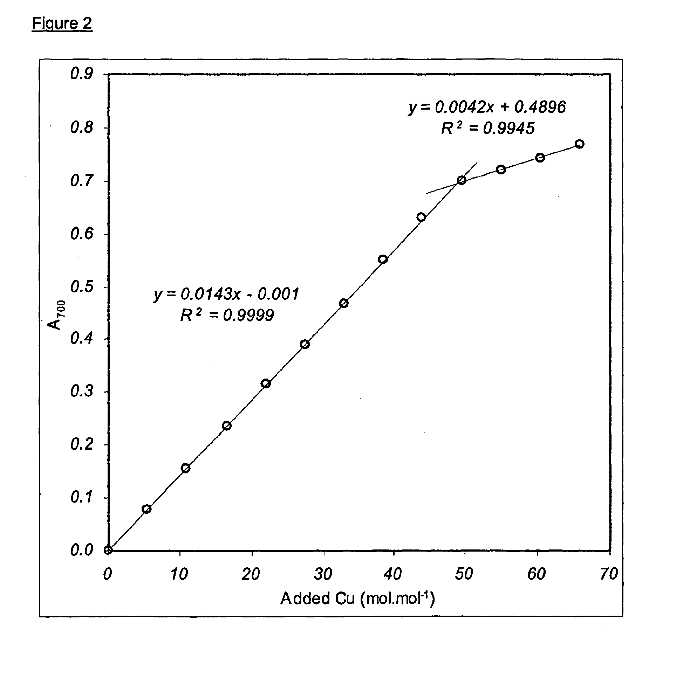

As can be seen in Figure 2, the amount of copper binding (taken as the intersect) closely matched the figure obtained for the gadolinium binding (determined by precipitation endpoint, as described in Example 1 above)

Example 3

Optimisation of carrier loading

For maximal effect, it is important in many cases that the carrier carries the maximal load of the agent, for example $Gd^{3+}$ for use in MRI, or radioactive metal for use in nuclear imaging or therapy. This method describes the optimisation of $Gd^{3+}$ binding capacity for rHA.

3.1 Synthesis of rHA-DTPA

Four 0.6g aliquots of rHA were each diluted to 24mL in water. 2, 1, 0.5 or 0.2g DTPAa were added to each over 25, 20, 12 and 9min respectively with constant stirring, the pH being maintained as close as possible to pH8 by addition of 5M NaOH. The solutions were stirred for ≈30min after the final addition and adjusted to pH7 with 3M HCl. All samples were dialysed together for a total of 44h against 4 changes of 11 L water.

3.2 Measurement of $Gd^{3+}$ binding capacity

$Gd^{3+}$ binding capacity was determined by complexometric titration with XO indicator, which, at around pH6, changes from yellow to purple in the presence of free $Gd^{3+}$. Because XO colour is also dependent on pH and large pH changes were found to occur during $GdCl_3$ titration of rHA-DTPA in unbuffered solution, good pH control was required during the titration for reliable results.

1 mL of each rHA-DTPA sample was mixed with 0.2mL 0.2M hexamine and 5$\mu$L 0,1%(w/v) XO (start pH5.9-6.1) and titrated with 0.1M $GdCl_3$ to the first permanent colour change (end pH5.4-5.6). To confirm the validity of the results, each sample was also subjected to spectrophotometric titration with $CuSO_4$, as in Example 2. Both titrants were standardised against a Standard Volumetric Solution of 10mM EDTA disodium salt (Fisher).

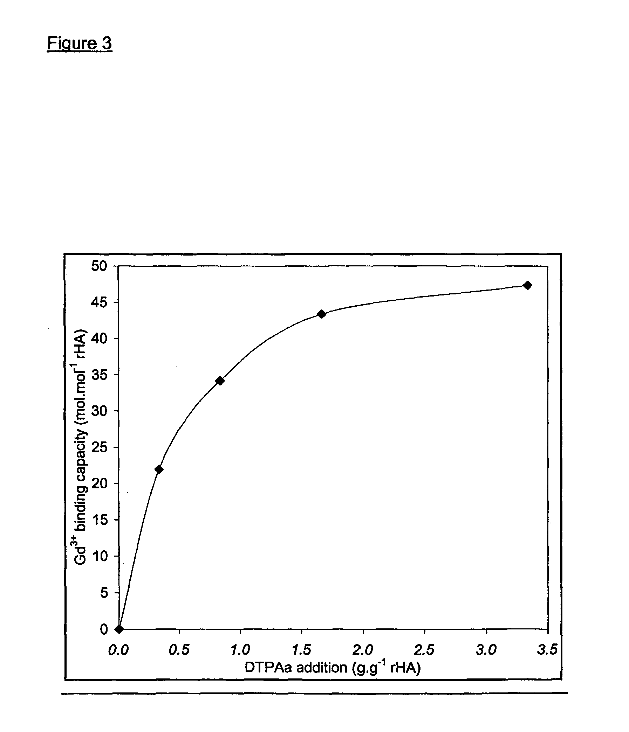

The results (Table 1 and Figure 3) showed excellent agreement between the two methods, confirming that both were reliable measures of the available DTPA groups. They also indicated that the highest level of DTPAa addition (3.33g DTPAa.g$^{-1}$ rHA; equivalent to 10mol DTPAa.mol$^{-1}$ rHA-NH$_2$) was required to give essentially maximal $Gd^{3+}$ binding capacity.

Table 1

| DTPAa addition (g.g$^{-1}$ rHA) | End point (mol.mol$^{-1}$ rHA) | |
|---|---|---|
| | Gd titration | Cu titration |
| 3.33 | 47.3 | 47.3 |
| 1.67 | 43.4 | 43.0 |
| 0.83 | 34.2 | 34.3 |
| 0.33 | 21.9 | 20.1 |

Example 4

Optimisation of removal of excess reactants

After labelling of the carrier molecule, it is desirable to remove excess reactants. The use of dialysis for this purpose was found to require extremely long times and extremely large volumes to achieve efficient removal (see, for instance, Example 3.1). The use of gel filtration offers dramatic savings in time and volume. This method describes the development of gel filtration for removal of excess DTPA from rHA-DTPA.

4.1 Synthesis of rHA-DTPA

0.3g rHA was diluted to 12mL in water and 1g DTPAa added over ≈30min with constant stirring, the pH being maintained as close as possible to pH8 by addition of 5M NaOH. The solution was stirred for =60min after the final addition and adjusted to pH7 with 3M HCl. Free thiol assay of this material gave a value of 0.10mol.mol$^{-1}$ rHA, compared to a value of 0.68mol.mol$^{-1}$ rHA for the starting rHA.

4.2 Sephadex G25 chromatography

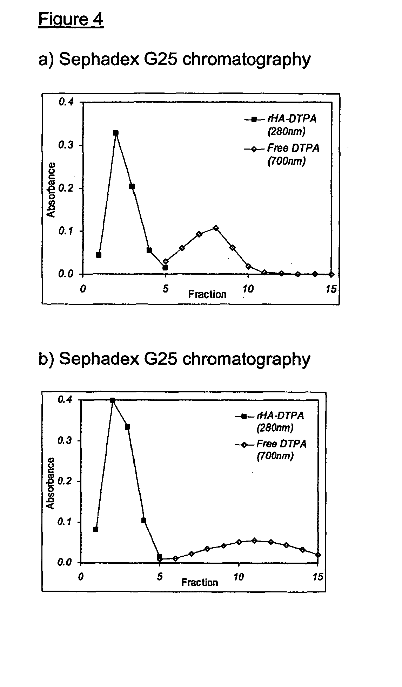

0.5mL rHA-DTPA was loaded onto a PD10 column (a pre-packed 8.3mL column of Sephadex G25 medium from Amersham Biosciences), equilibrated in 0.9%(w/v) NaCl. The column was eluted with 0.5mL aliquots 0.9%(w/v) NaCl, the first four to waste and the remainder collected for assay. To determine the elution profile of the rHA-DTPA, 0.1mL of the appropriate fractions was diluted with 1mL water and the absorbance at 280nm measured. To determine the elution profile of the excess free DTPA, 20μL of the appropriate fractions was diluted with 1mL 4mM CuSO$_4$ in 20mM hexamine pH5 and the absorbance at 700nm measured. The results (Figure 4a) indicated that baseline separation of rHA-DTPA from excess free DTPA was not achieved despite the relatively low column loading (6% column volume).

4.3 Sephadex G25 chromatography (small scale)

A PD10 column was emptied and repacked to the same bed volume with Sephadex G50 medium (Amersham Biosciences). The column was equilibrated in 0.9%(w/v) NaCl and 0.5mL rHA-DTPA was loaded. The column was eluted and the fractions assayed as above. The results (Figure 4b) indicated a marked improvement in the separation of the two peaks with the higher porosity matrix, even though the self-packed column was less efficient than the commercially packed one, resulting in significantly greater peak widths.

4.4 Detection wavelength

An absorbance spectrum of 0.18M DTPA pH7 (the approximate concentration and pH at the end of the rHA/ DTPAa reaction) showed negligible absorbance above 270nm with significant absorbance appearing below 260nm. A detection wavelength of 254nm was therefore chosen for monitoring the column effluent to allow detection of both rHA-DTPA and free DTPA.

4.5 Sephadex G50 chromatography (large scale)

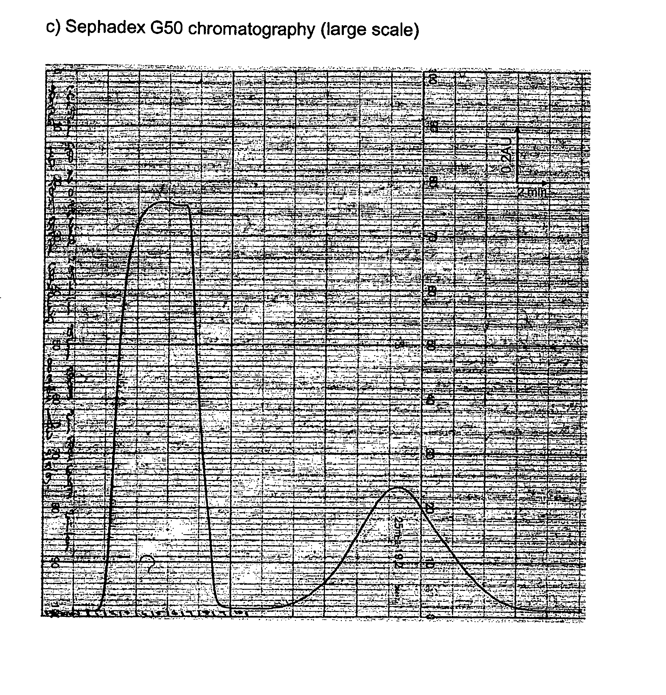

An XK16/40 column (Amersham Biosciences) was packed with Sephadex G50 medium in 0.9%(w/v) NaCl to a bed height of 37cm and connected to a UV-1 monitor with 2mm flow cell (Amersham Biosciences) set to 254nm and 2AU full scale. A fresh sample of rHA-DTPA was prepared as above, but with stirring time between the final addition and adjustment to pH7 reduced to ≈30min. This material was loaded onto the column and eluted with 0.9%(w/v) NaCl at 2.85mL.min$^{-1}$. The results (Figure 4c) indicated that, even with the high column loading (20% column volume), baseline separation of rHA-DTPA from excess free DTPA was easily achieved, with the purified rHA-DTPA being produced within 10min from the start of column elution.

Example 5

Development of conditions for free thiol recovery in soluble rHA derivatives

Previous results (see Example 4) indicated that the rHA free thiol was largely blocked by the reaction with DTPAa. If this thiol was to be used as the site of cross-linker attachment, recovery of the rHA free thiol was required for efficient reaction with the cross-linker. This method describes the development of conditions to achieve this aim, either following reaction of rHA with DTPAa (rHA-DTPA) or the binding of Gd$^{3+}$ to the rHA-DTPA (rHA-DTPA-Gd).

5.1 Effect of storage on rHA-DTPA free thiol

The free thiol content of the rHA-DTPA from Example 4 was reassayed after 6 weeks refrigerated storage and found to have increased from 0.10 to 0.29mol.mol$^{-1}$ rHA. This suggested that recovery of the free thiol might be possible under suitable conditions.

### 5.2 Synthesis of rHA-DTPA

Synthesis of rHA-DTPA was performed as in Example 4 Sephadex G50 chromatography (large scale) with the rHA-DTPA peak collected from 2 to 9min after the start of elution. The free thiol level in the starting rHA was $0.70 \text{mol.mol}^{-1}$ rHA.

### 5.3 Synthesis of rHA-DTPA-Gd

9.8mL rHA-DTPA was mixed with $30\mu L$ 0.1%(w/v) XO and titrated with 0.1 M $GdCl_3$, pH being maintained between pH5.5 and pH6.0 with 1 M NaOH, to the end point ($\equiv 48 \text{mol Gd}^{3+}.\text{mol}^{-1}$ rHA), indicated by the first change from yellow to purple.

A further 0.1mL rHA-DTPA was added to bind surplus $Gd^{3+}$, returning the colour to yellow, and the solution was adjusted to pH7 with 1 M NaOH.

### 5.4 Recovery of free thiol

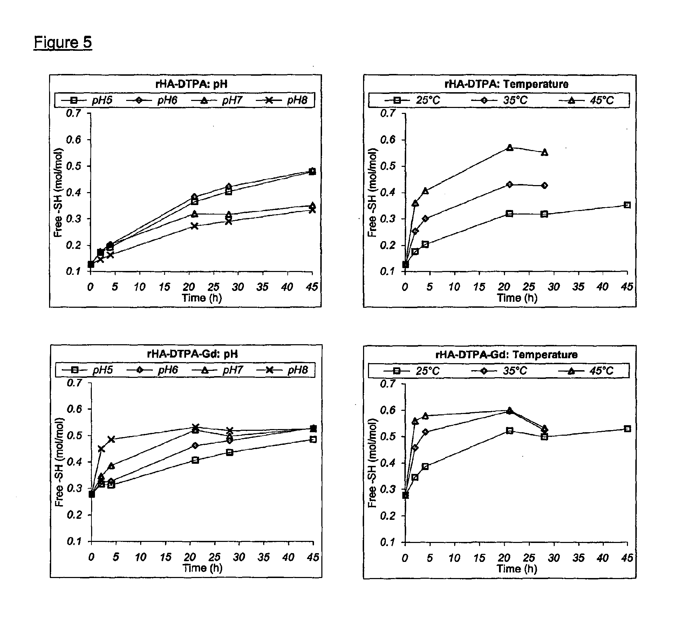

Six 1mL aliquots of both rHA-DTPA and rHA-DTPA-Gd were taken. Three of each type were adjusted to either pH5, pH6 or pH8 with HCl or NaOH as appropriate and the remaining three left at pH7. All samples were made up to 1.1 mL with water. Two pH7 samples of each type were incubated at either 35˚C or 45˚C and the remaining samples all incubated at 25˚C. Free thiol assays were performed on the starting rHA-DTPA and rHA-DTPA-Gd and then on each of the incubations at the indicated times. The results (Figure 5) indicated that the free thiol in both samples could be recovered by simple incubation, although the rate of recovery was much higher with rHA-DTPA-Gd than with rHA-DTPA. Both reactions showed some pH dependency, although increasing temperature was more effective at increasing reaction rate. Maximal recovery (equivalent to approximately 80% starting free thiol) was achieved at 45˚C in around 2h for rHA-DTPA-Gd and 20h for rHA-DTPA.

### Example 6

### Cross-linker reaction with soluble rHA derivatives

In the methodology according to the invention the targeting moiety and the carrier molecule each contain distinct chemical groups for selective cross-linker reaction to produce a well-defined product. For the particular case of an antibody targeting moiety and a rHA carrier molecule, these groups are preferably carbohydrate and free thiol respectively. This method describes the reaction of rHA-DTPA and rHA-DTPA-Gd with two cross-linkers, PDPH and EMCH, suitable for use with these groups.

### 6.1 Synthesis of rHA-DTPA

0.3g rHA (free thiol level $0.67 \text{mol.mol}^{-1}$ rHA) was diluted to 12mL in water and 1g DTPAa added over $\approx 30$min with constant stirring, the pH being maintained as close as possible to pH8 by addition of 5M NaOH. The solution was stirred for =30min after the final addition, adjusted to pH7 with 3M HCl and applied to a Sephadex G50 medium column (1.6×37cm) equilibrated in 0.9%(w/v) NaCl. Elution was performed at $2.84 \text{mL.min}^{-1}$ with detection at 254nm. The rHA-DTPA peak was collected from 2 to 9min after the start of elution and showed a free thiol level of $0.07 \text{mol.mol}^{-1}$ rHA. The rHA-DTPA was incubated for 20h at 45˚C to unblock the thiol, yielding a free thiol level of $0.57 \text{mol.mol}^{-1}$ rHA.

### 6.2 Synthesis of rHA-DTPA-Gd

0.3g rHA (free thiol level $0.70 \text{mol.mol}^{-1}$ rHA) was converted to rHA-DTPA as above, except that a) DTPAa was added over $\approx 35$min; b) the Sephadex G50 column was eluted at $2.7 \text{mL.min}^{-1}$; c) the rHA-DTPA peak was collected from 2 to 10min after the start of elution and showed a free thiol level of $0.14 \text{mol.mol}^{-1}$ rHA. 1 mL rHA-DTPA was removed and $60\mu L$ 0.1%(w/v) XO added to the remainder. The DTPA groups were titrated with 0.1 M $GdCl_3$, pH being maintained between pH5.5 and pH6.0 with 1 M NaOH, to the end point ($\equiv 48 \text{mol Gd}^{3+}.\text{mol}^{-1}$ rHA). 0.3mL retained rHA-DTPA was added to bind surplus $Gd^{3+}$, the solution adjusted to pH7 with 1M NaOH and then incubated for 2h at 45˚C to unblock the thiol, yielding a free thiol level of $0.51 \text{mol.mol}^{-1}$ rHA.

6.3 Reaction with PDPH

2mL rHA-DTPA was mixed with 0.15mL 0.2M $Na_2HPO_4$ and 0.05mL 0.2M $NaH_2PO_4$. A 1 mL sample was taken and its absorbance at 343nm measured immediately before addition of 0.1mL 10mM PDPH (Pierce) and every 2min thereafter for 20min. Measurements were corrected for the absorbance of the PDPH itself and the extent of reaction calculated using an extinction coefficient of $8080M^{-1}.cm^{-1}$ for the released 2-thiopyridine. The experiment was repeated with rHA-DTPA-Gd. The results indicated that the reaction with PDPH was both rapid and efficient in both cases, being 96-98% complete at the first 2min time point, with the final extent of reaction being =90% of that measured by the free thiol assay.

6.4 Reaction with EMCH

4mL rHA-DTPA-Gd was mixed with 0.3mL 0.2M $Na_2HPO_4$ and 0.1 mL 0.2M $NaH_2PO_4$ and 0.44mL 10mM EMCH (Pierce) added. 1 mL samples were taken at 15, 30, 60 and 120min after addition and immediately applied to a PD10 column equilibrated in 50mM sodium phosphate pH7 to remove the excess EMCH. The high molecular weight fraction was collected from 1.5 to 3.5mL after the start of elution. Eluates were subjected to free thiol assay, both directly and spiked with a constant amount of DTT (to evaluate EMCH removal) and to protein assay (to evaluate rHA recovery). Measured recoveries were in the range 98-104% for DTT and 102-104% for rHA, confirming complete elimination of EMCH and recovery of rHA. The results for the direct free thiol assay indicated that the reaction with EMCH was both rapid and efficient, with free thiol level being reduced to $0.02mol.mol^{-1}$ rHA at the first 15min time point.

Example 7

Cross-linker reaction with antibodies

This Example describes the reaction of the two antibodies $\alpha$CD45 and C595 with the cross-linker PDPH.

7.1 $\alpha$CD45 reaction with PDPH

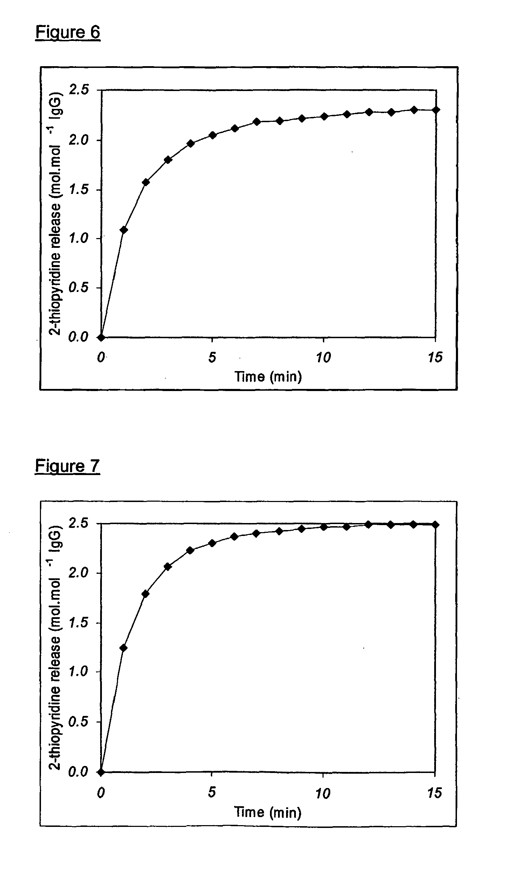

1mL $\alpha$CD45 (Serotec MCA43G; IgG concentration $1.0mg.mL^{-1}$) was added to 2.3mg $KIO_4$ and mixed for 30min at room temperature in the dark. The solution was applied to a PD10 column equilibrated in PBS and the high molecular weight fraction collected from 1.5 to 3.5mL after the start of elution. The eluate was concentrated to a final volume of 1mL, using a Nanosep 10K Omega centrifugal concentrator (Pall) pre-washed with PBS, added to 1.0mg PDPH and mixed for 21 h at room temperature. $\alpha$CD45-PDPH was purified using a PD10 column and the eluate reconcentrated to 1mL as above, giving an overall IgG recovery of 90%. The extent of reaction was determined from the 2-thiopyridine released on reduction of the bound PDPH, by absorbance measurement at 343nm immediately before addition of 10$\mu$L 10mM DTT and every 1 min thereafter for 15min, using an extinction coefficient of $8080M^{-1}.cm^{-1}$. The results (Figure 6) indicated that PDPH reacted successfully with $\alpha$CD45, giving a stoichiometry of 2.3mol PDPH.mol$^{-1}$ IgG. Analytical GPHPLC (50$\mu$L injection with detection wavelength of 280nm) indicated that the resulting $\alpha$CD45-PDPH retained high monomeric purity, with a peak elution time of =8.4min.

7.2 C595 reaction with PDPH

0.35mL C595 (obtained from The University of Nottingham; IgG concentration $3.2mg.mL^{-1}$) was diluted with 0.65mL PBS, added to 2.4mg $KIO_4$ and mixed for 30min at room temperature in the dark. The solution was applied to a PD10 column equilibrated in PBS and the high molecular weight fraction collected from 1.5 to 3.5mL after the start of elution. The eluate was concentrated to a final volume of 1 mL, using a Nanosep 10K Omega centrifugal concentrator pre-washed with PBS, added to 1.0mg PDPH and mixed for 19h at room temperature. C595-PDPH was purified using a PD10 column and the eluate reconcentrated to 1mL as above, giving an overall IgG recovery of 85%. The extent of reaction was determined from the 2-thiopyridine released on reduction of the bound PDPH, by absorbance measurement at 343nm immediately before addition of 10$\mu$L 10mM DTT and every 1 min thereafter for 15min, using an extinction coefficient of $8080M^{-1}.cm^{-1}$. The results (Figure 7) indicated that PDPH reacted successfully with C595, giving a stoichiometry of 2.5mol PDPH.mol$^{-1}$ IgG. Analytical GPHPLC (50$\mu$L injection with detection wavelength of 280nm) indicated that the resulting C595-PDPH retained high monomeric purity, with a peak elution time of $\approx$8.5min.

Example 8

Purification of rHA-DTPA/rHA-DTPA-Gd

The conjugate of the targeting moiety and the carrier molecule will clearly be larger than either of the individual components and hence purification of the conjugate from any of the unreacted individual components should be achievable by gel permeation chromatography. For the particular case of a rHA carrier molecule, the tendency of human albumin to form dimers and higher oligomers could compromise the successful purification of the targeted conjugate away from non-targeted carrier molecules. This method describes the purification of monomeric rHA-DTPA and rHA-DTPA-Gd, prior to reaction with the targeting moiety, to simplify the subsequent purification of the conjugate.

8.1 Preparative GPHPLC

Purification of rHA derivatives used a large injection volume (200μL), to give high productivity, and a sub-optimal detection wavelength (254nm), to reduce peak absorbance at high rHA concentration.

8.2 Purification of rHA-DTPA

Preparative GPHPLC of rHA-DTPA was characterised by a dimer peak at ≈6.6min and a monomer peak at =7.6min. For purification of monomeric rHA-DTPA, material eluting between 7.2 and 9.0min was collected. Analytical GPHPLC (50μL injection with detection wavelength of 280nm) of the collected material confirmed that a high degree of monomeric purity had been achieved by this method.

8.3 Purification of rHA-DTPA-Gd

Preparative GPHPLC of rHA-DTPA-Gd was characterised by a dimer peak at ≈6.9min and a monomer peak at ≈8.1 min. For purification of monomeric rHA-DTPA, material eluting between 7.7 and 9.0min was collected. Analytical GPHPLC (100μL injection with detection wavelength of 254nm) of the collected material confirmed that a high degree of monomeric purity had been achieved by this method.

Example 9

Preparation of αCD45-PDPH-rHA-DTPA-Gd

Based on the previous developments of individual steps in the synthesis (Examples 1-8), this method describes the complete preparation of a targeted agent, in which the carrier molecule (rHA) is labelled at high levels with DTPA followed by Gd, making it suitable for use as a targeted MRI contrast agent.

9.1 Synthesis of rHA-DTPA

0.3g rHA (free thiol level $0.70mol.mol^{-1}$ rHA) was diluted to 12mL in water and 1g DTPAa added over ≈35min with constant stirring, the pH being maintained as close as possible to pH8 by addition of 5M NaOH. The solution was stirred for 30min after the final addition, adjusted to pH7 with 3M HCl and applied to a Sephadex G50 medium column (1.6×37cm) equilibrated in 0.9%(w/v) NaCl. Elution was performed at $2.7mL.min^{-1}$ with detection at 254nm. The rHA-DTPA peak was collected from 2 to 10min after the start of elution and showed a free thiol level of $0.14mol.mol^{-1}$ rHA.

9.2 Synthesis of rHA-DTPA-Gd

1 mL rHA-DTPA was removed and 60μL 0.1%(w/v) XO added to the remainder. The DTPA groups were titrated with 0.1M $GdCl_3$, pH being maintained between pH5.5 and pH6.0 with 1 M NaOH, to the end point (≡$48mol\ Gd^{3+}.mol^{-1}$ rHA), indicated by the first change from yellow to purple. 0.3mL retained rHA-DTPA was added to bind surplus $Gd^{3+}$, returning the colour to yellow, the solution adjusted to pH7 with 1 M NaOH and then incubated for 2h at 45°C to unblock the thiol, yielding a free thiol level of $0.51mol.mol^{-1}$ rHA.

9.3 Synthesis of αCD45-PDPH

1 mL αCD45 was added to 2.3mg $KIO_4$ and mixed for 30min at room temperature in the dark. The solution was applied to a PD10 column equilibrated in PBS and the high molecular weight fraction collected from 1.5 to 3.5mL after the start of elution. The eluate was concentrated to a final volume of 1mL, using a Nanosep 10K Omega centrifugal concentrator pre-washed with PBS, added to 1.1mg PDPH and mixed for 5h at room temperature. αCD45-PDPH was purified using a PD10 column, run as above.

9.4 <u>Purification of rHA-DTPA-Gd</u>

Monomeric rHA-DTPA-Gd was purified by preparative GPHPLC using a 200μL injection with detection at 254nm. Eight cycles of chromatography were performed, with product collection from 7.7-9.0min after injection. The rHA concentration of the product was $1.1g.L^{-1}$. High monomeric purity of the product was confirmed by analytical GPHPLC using a 50μL injection with detection at 280nm.

9.5 <u>Synthesis of αCD45-PDPH-rHA-DTPA-Gd</u>

Purified rHA-DTPA-Gd was added to the αCD45-PDPH at ≈10mol rHA.mol⁻¹ IgG, the solution concentrated to 1mL, using a Vivaspin20 10K centrifugal concentrator (Sartorius) pre-washed with PBS, and mixed for 24h at room temperature. The formation of αCD45-PDPH-rHA-DTPA-Gd was confirmed by the appearance of a new high molecular weight peak on analytical GPHPLC using a 20μL injection with detection at 280nm. An assumed rHA:αCD45 stoichiometry of 2.3 (as measured in Example 7 for PDPH:αCD45) together with the measured $Gd^{3+}$:rHA stoichiometry of 48 gives a predicted Gd level for this complex of ≈10mol $Gd^{3+}.mol^{-1}$ IgG.

9.6 <u>Purification of αCD45-PDPH-rHA-DTPA-Gd</u>

αCD45-PDPH-rHA-DTPA-Gd was purified by preparative GPHPLC as above but using ten injections of≈95μL, detection at 280nm and product collection from 6.0-7.2min. The product was concentrated to 1 mL using two Nanosep 10K Omega centrifugal concentrators and purity confirmed by analytical GPHPLC using a 50μL injection with detection at 280nm. Finally, the product was frozen in ≈150μL aliquots in a -30˚C bath and stored at -20˚C.

9.7 <u>Antibody binding activity</u>

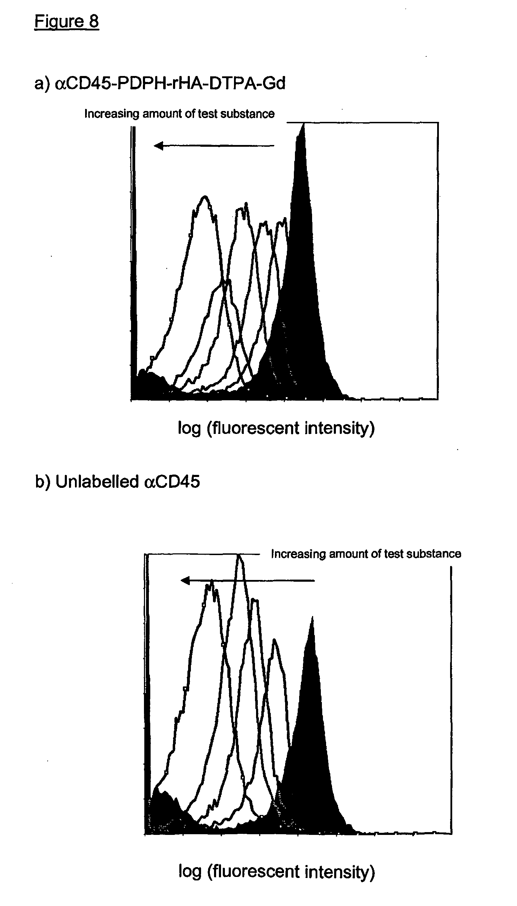

Antibody activity of αCD45-PDPH-rHA-DTPA-Gd was measured using rat splenocytes, which express the leukocyte common antigen CD45, in a competitive binding assay against fluorophore-labelled αCD45. The fluorescent intensity of the cells was measured by flow cytometry, using a constant 0.2μg fluorescent antibody and increasing amounts of αCD45-PDPH-rHA-DTPA-Gd. Unlabelled αCD45 was assayed similarly as a positive control. The results (Figure 8) indicated that αCD45-PDPH-rHA-DTPA-Gd was able to displace the fluorescent antibody in the same manner as the un labelled αCD45, demonstrating that the conjugate retained antibody binding activity.

9.8 <u>Magnetic resonance properties</u>

αCD45-PDPH-rHA-DTPA-Gd gave T1 and T2 relaxation times at 2Tesla of 92 and 95ms respectively. This was approximately two-fold better than 0.5mM Omniscan™ (a non-targeted gadolinium based MRI contrast agent from Amersham) which, measured simultaneously, gave values of 193 and 177ms, indicating excellent relaxation enhancement for the conjugate.

<u>Example 10</u>

<u>Preparation of C595-PDPH-rHA-DTPA</u>

This method describes the preparation of a targeted agent, in which the carrier molecule (rHA) is labelled at high levels with DTPA but no metal ion, making it suitable for loading with an appropriate radioactive metal for use as a targeted nuclear imaging or therapeutic agent.

10.1 <u>Synthesis of rHA-DTPA</u>

0.3g rHA (free thiol level 0.67mol.mol⁻¹ rHA) was diluted to 12mL in water and 1g DTPAa added over ≈30min with constant stirring, the pH being maintained as close as possible to pH8 by addition of 5M NaOH. The solution was stirred for 30min after the final addition, adjusted to pH7 with 3M HCl and applied to a Sephadex G50 medium column (1.6×37cm) equilibrated in 0.9%(w/v) NaCl. Elution was performed at 2.84mL.min⁻¹ with detection at 254nm. The rHA-DTPA peak was collected from 2 to 9min after the start of elution and showed a free thiol level of 0.07mol.mol⁻¹ rHA.

The rHA-DTPA was incubated for 20h at 45˚C to unblock the thiol, yielding a free thiol level of 0.57mol.mol$^{-1}$ rHA. The DTPA level, determined on a 4mL aliquot of this material by complexometric titration with $GdCl_3$ as above, was 44mol.mol$^{-1}$ rHA.

## 10.2 Synthesis of C595-PDPH

C595 (obtained from The University of Nottingham; IgG concentration 3.2mg.mL$^{-1}$) was diluted to 1.0mg.mL$^{-1}$ IgG in PBS, 1mL added to 2.3mg $KIO_4$ and mixed for 30min at room temperature in the dark. The solution was applied to a PD10 column equilibrated in PBS and the high molecular weight fraction collected from 1.5 to 3.5mL after the start of elution. The eluate was concentrated to a final volume of 1 mL, using a Nanosep 10K Omega centrifugal concentrator pre-washed with PBS, added to 1.1mg PDPH and mixed for 5h at room temperature. C595-PDPH was purified using a PD10 column, run as above.

## 10.3 Purification of rHA-DTPA

Monomeric rHA-DTPA was purified by preparative GPHPLC using a 200μL injection on a TSKgel G3000SWXL 0.78x30cm column and guard, eluted at 1 mL.min$^{-1}$ in PBS with detection at 254nm. Six cycles of chromatography were performed, with product collection from 7.2-9.0min after injection. The rHA concentration of the product was 1.0g.L$^{-1}$. High monomeric purity of the product was confirmed by analytical GPHPLC, performed as preparative GPHPLC above but using a 50μL injection with detection at 280nm.

## 10.4 Synthesis of C595-PDPH-rHA-DTPA

Purified rHA-DTPA was added to the C595-PDPH at ≈10mol rHA.mol$^{-1}$ IgG, the solution concentrated to 1mL, using a Vivaspin20 10K centrifugal concentrator pre-washed with PBS, and mixed for 18h at room temperature. The formation of C595-PDPH-rHA-DTPA was confirmed by the appearance of a new high molecular weight peak on analytical GPHPLC using a 20μL injection with detection at 280nm. An assumed rHA:C595 stoichiometry of 2.5 (as measured in Example 7 for PDPH:C595) together with the measured $Gd^{3+}$:rHA stoichiometry of 44 gives a predicted Gd level for this complex of ≈110mol $Gd^{3+}$.mol$^{-1}$ IgG.

## 10.5 Purification of C595-PDPH-rHA-DTPA

C595-PDPH-rHA-DTPA was purified by preparative GPHPLC as above but using ten injections of ≈95μL, detection at 280nm and product collection from 5.7-6.6min. The product was concentrated to 1 mL using two Nanosep 10K Omega centrifugal concentrators and purity confirmed by analytical GPHPLC using a 50μL injection with detection at 280nm. Finally, the product was frozen in ≈115μL aliquots in a -30˚C bath and stored at -20˚C.

## Example 11

## Preparation of rHA particles labelled with MRI contrast agent (gadolinium chelate)

### 11.1 Methods

### 11.1.1 Preparation of rHA particles

A batch of rHA particles was produced by spray drying of a rHA suspension, as follows:

200mL 25%(w/v) rHA was dialysed against 5L pyrogen-free purified water overnight at room temperature to remove excess sodium chloride, giving a final volume of 320mL. 52mL dialysed protein was adjusted to pH8.0 with 1 M NaOH and ethanol added slowly with constant mixing on a magnetic stirrer until a milky suspension was formed (80mL ethanol). The continuously agitated suspension was spray dried using a Buchi Mini Spray Dryer (model B-191), fitted with Schlick 2-fluid atomisation nozzle (model 970/0), under the following conditions:

inlet temperature 100˚C
outlet temperature 67˚C
feed rate 3mL.min$^{-1}$
atomisation pressure 6barg

The resulting microparticles (4.0g) were recovered from the cyclone collection jar, heat-fixed at 176˚C for 55min to render them insoluble (yielding 3.5g), mixed with 7.0g mannitol and deagglomerated using an Attritor 6in fluid energy mill with an inlet pressure of 5barg and a milling pressure of 3barg.

### 11.1.2 Conjugation with DTPAa

The formulated particles were wetted with ethanol and washed thoroughly with water by centrifugation (Sorvall RT6000) to remove the excipient mannitol. After washing, an aliquot was taken for dry weight determination and the rHA particles diluted to 20g.L$^{-1}$ (based on this measurement) in water. DTPAa (1g per 0.3g particles) was added slowly with constant stirring over a period of ≈25min. During this time, 5M NaOH was added to maintain the pH as close to 8.0 as possible. Stirring was continued for 30min after the final DTPAa addition and the pH then adjusted to 7.0 with 5M HCl. The rHA-DTPA particles were washed twice with 0.9%(w/v) NaCl and twice with water by centrifugation and resuspension to 8g.L$^{-1}$ (based on the initial dry weight) and finally resuspended to 60g.L$^{-1}$.

### 11.1.3 Labelling with Gd

The dry weight of the rHA-DTPA particle suspension was measured to determine the mass increase due to DTPA labelling, giving a value of 32.4mol.mol$^{-1}$ rHA. For Gd labelling, a further aliquot of the suspension was diluted with water, and 0.1 M GdCl$_3$ added with constant mixing to give an overall dilution of 2.5-fold and a final Gd concentration equal to the DTPA level determined by dry weight measurement. Mixing was continued for 10min, the suspension diluted a further 1.5-fold with water, the rHA-Gd particles sedimented by centrifugation, and the pellet resuspended in water to 60g.L$^{-1}$ (based on the initial dry weight). The resulting suspension was formulated to 50g.L$^{-1}$, 5%(w/v) mannitol and freeze dried.

### 11.2 Characterisation of Particles

### 11.2.1 Magnetic Resonance Properties of the Particles

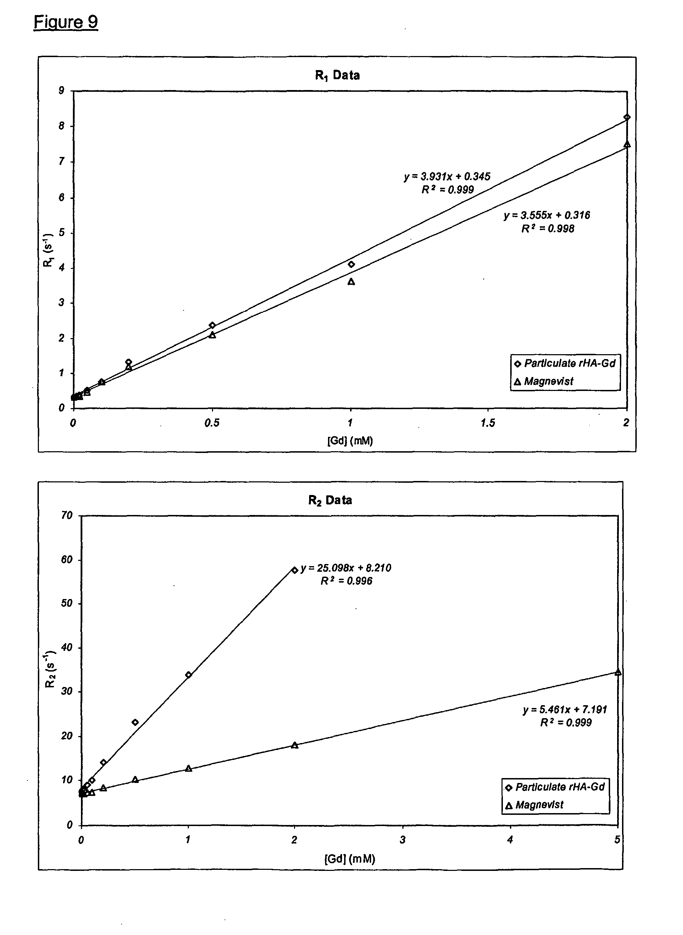

Magnetic resonance imaging was performed on the Gd-DTPA labelled particles. The particles and the Magnevist™ control were diluted appropriately to 2mL with water, and then mixed with 8mL 0.5% agarose to give the specified Gd concentration. Relaxation rates (R1 and R2) for each sample were determined, as described in Example 1.
The *in vitro* MRI properties of rHA-Gd particles were compared against those of Magnevist™, a commercial MRI contrast agent based on Gd-DTPA. Relaxation rates with the particulate rHA-Gd (Figure 9) were equivalent to (for R1) or significantly greater than (for R2) those obtained with Magnevist™. This indicated that rHA-Gd particles should produce *in vivo* images equivalent to or better than those seen with Magnevist™ at the same Gd concentration, particularly in view of the marked reduction in clearance rate anticipated for the particulate material. Alternatively, it suggested that the particles could be used at a lower Gd dose than Magnevist™.

### 11.2.2 Size Distribution Analysis

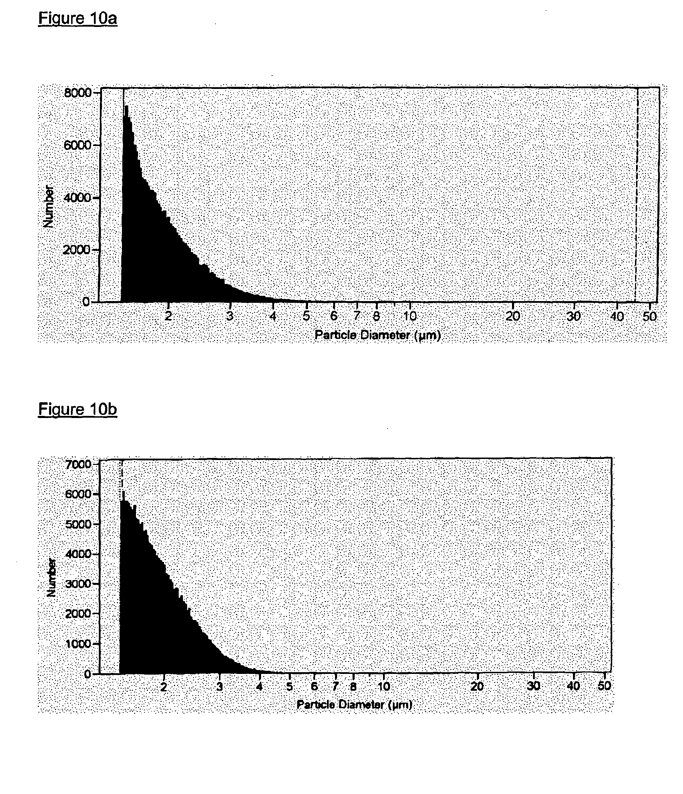

Coulter size analysis was undertaken to determine whether the labelling method had led to significant levels of agglomeration. The results shown in Figure 10 confirm that the size distribution (post Gd-DTPA labelling) was not significantly changed.

### 11.2.3 Resuspension properties

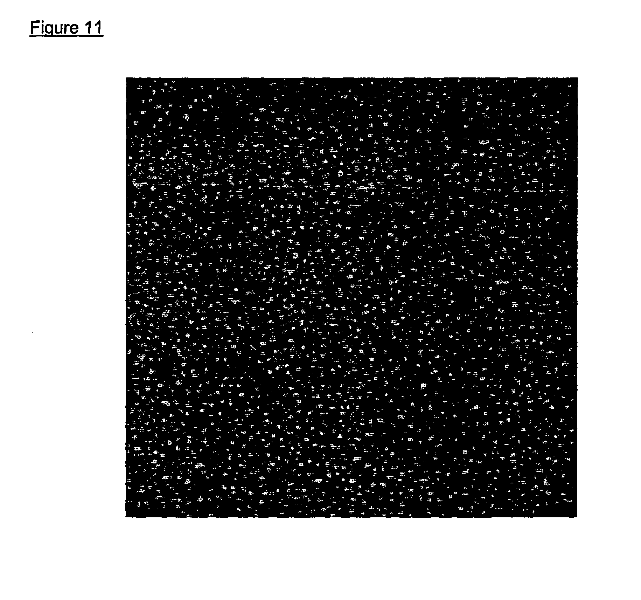

Light microscopy (Figure 11) confirmed that the Gd-DTPA labelled particles were non-agglomerated and resuspended to give a suitable suspension.

### Example 12

### Labelling soluble rHA with an MRI contrast agent and then making particles

This Example illustrates a variation on the methodology of the invention, in which particles are formed from soluble rHA molecules that have previously been labelled with a Gd-chelate.

### 12.1 Methods

A 1.5g aliquot of rHA was diluted to 20g.L$^{-1}$ in water and treated with 5g DTPAa, added slowly with constant stirring over a period of approximately 40min. During this time, 5M NaOH was added to maintain the pH as close to 8.0 as possible. Stirring was continued for 30min after the final DTPAa addition and the pH then adjusted to 7.0 with 5M HCl.

The labelled (+DTPA) sample was dialysed against 10.5L water, changed after 5h, for a total of 21 h. To estimate the required Gd addition, a 5mL aliquot of the +DTPA sample was titrated with 1 M $GdCl_3$ to the first permanent haze. On this basis, the DTPA level obtained with the soluble rHA was 47mol.mol$^{-1}$. A further 5mL aliquot of the +DTPA sample was removed, the remaining sample titrated with 1M $GdCl_3$ to the first permanent haze and the untitrated aliquot added back to bind any excess Gd.

The Gd-labelled sample was then 0.2$\mu$m filtered to remove any residual insoluble material. The samples were spray dried using a Buchi spray drier with Schlick two fluid nozzle at an inlet temperature of 110°C, an atomisation pressure of 0.5bar and a feed rate of 3.5mL.min$^{-1}$, giving an outlet temperature of approximately 79°C. The collected sample was weighed, to calculate recovery, and then heat-fixed for a total of 5½hour at 175°C.

In the absence of DTPA labelling, rHA particles are insoluble after 55 minutes at 175°C. However, this was not the case with the Gd-labelled sample and additional fixation was required to achieve insolubility of the material.

## Example 13

### Free thiol content of particulate rHA

For use as a targeted agent, it is important that the particles contain a suitable reactive group for chemical coupling of the targeting moiety. In the case of rHA as the particle-forming material, the free thiol at cys34 is a highly suitable group for this purpose. This method describes the measurement of the free thiol content of rHA particles, to confirm that it is still available for reaction, despite the high-temperature heat-fixation used to render the particles insoluble.

### 13.1 Preparation of rHA particles

100mL 25%(w/v) rHA was dialysed overnight at room temperature against 20L water and the resulting protein concentration determined by absorbance measurement at 280nm, using a 1g.L$^{-1}$ extinction coefficient of 0.53. The dialysed protein (24.4g) was diluted with water to 12.5%(w/v) and adjusted to pH8.0 with NaOH. 275mL ethanol was added and the resulting suspension spray-dried with an inlet temperature of 100°C, outlet temperature of 72°C, a flow rate of 3mL.min$^{-1}$ and an atomisation pressure of 6barg. The resulting microparticles (13.4g) were heat-fixed at 175°C for 1 h (yielding 11.1g), mixed with 22.2g mannitol and passed twice through the fluid energy mill with an inlet pressure of 5barg and a milling pressure of 3barg.

### 13.2 Free thiol assay

The free thiol concentration was determined by reaction with DTNB at pH7 and absorbance measurement at 412nm, using an extinction coefficient of 13600M$^{-1}$.cm$^{-1}$ for the released 5-thio-2-nitrobenzoate. Reaction was performed for 1 h at room temperature and the particles sedimented by centrifugation prior to absorbance measurement on the supernatant. Correction was made both for the absorbance of the DTNB and the residual turbidity from unsedimented particles. The measured free thiol level was 0.36mol.mol$^{-1}$ rHA, confirming that the free thiol was not completely destroyed by the heat-fixation step and was present at a suitable level for coupling of a targeting moiety.

## Example 14

### Optimisation of particle loading

For maximal effect, it is important in many cases that the particle carries the maximal load of the agent, for example $Gd^{3+}$ for use in MRI, or radioactive metal for use in nuclear imaging or therapy. This method describes the optimisation of $Gd^{3+}$ binding capacity for rHA particles.

### 14.1 Synthesis of DTPA-labelled rHA particles

Aliquots of rHA particles are suspended in water and varying amounts of DTPAa (covering the range ≈0.5-5g.g$^{-1}$ rHA) are added to each over ≈10-40min with constant stirring, the pH being maintained as close as possible to pH8 by addition of 5M NaOH. The suspensions are stirred for ≈30min after the final addition and adjusted to pH7 with 3M HCl. All samples are washed thoroughly by centrifugation and resuspension to remove unbound DTPA.

14.2 Measurement of Gd$^{3+}$ binding capacity

Gd$^{3+}$ binding capacity is determined by complexometric titration with XO indicator. pH is controlled in the range pH5.0-6.5 during the titration, either by use of an appropriate buffer (eg hexamine) or by adjustment with an appropriate alkali (eg NaOH). XO is added to a suspension containing a known amount of DTPA-labelled rHA particles and titration performed with a standardised GdCl$_3$ solution to the first permanent colour change.

Example 15

Development of conditions for free thiol recovery in particulate rHA derivatives

Results with soluble rHA (see Example 4) indicate that the rHA free thiol is largely blocked by the reaction with DTPAa. If this thiol is to be used as the site of cross-linker attachment, recovery of the rHA free thiol is required for efficient reaction with the cross-linker. This method describes the development of conditions to achieve this aim, either following reaction of rHA particles with DTPAa (DTPA-labelled rHA particles) or following the binding of Gd$^{3+}$ (Gd-labelled rHA particles).

15.1 Synthesis of labelled rHA particles

DTPA-labelled rHA particles are produced essentially as described in Example 11. Gd-labelled rHA particles may be produced from the DTPA-labelled rHA particles essentially as described in Example 11. Alternatively, they may be produced using complexometric titration with GdCl$_3$ in the presence of XO, as described in Example 14, with a small further addition of DTPA-labelled rHA particles at the end of titration to bind surplus Gd$^{3+}$.

15.2 Recovery of free thiol

Aliquots of both DTPA-labelled and Gd-labelled rHA particles are incubated at a range of pH values and/or a range of temperatures and samples taken at the start of incubation and periodically thereafter. These are subjected to free thiol assay, to determine the optimum conditions for recovery of free thiol with each particle type.

Example 16

Cross-linker reaction with particulate rHA derivatives

In the methodology according to the invention the targeting moiety and the particle each contain a unique chemical group for cross-linker reaction to produce a well-defined product. For the particular case of an antibody targeting moiety and a rHA particle, these groups are carbohydrate and free thiol respectively. This method describes the reaction of DTPA-labelled rHA particles and Gd-labelled rHA particles with two cross-linkers, PDPH and EMCH, suitable for use with these groups.

16.1 Synthesis of labelled rHA particles

Both DTPA-labelled and Gd-labelled rHA particles are synthesised essentially as described in Example 15 and the free thiol unblocked using the optimum conditions determined in that Example.

16.2 Reaction with PDPH

Aliquots of both types of labelled rHA particles are buffered at around pH7 by addition of phosphate buffer. Samples are taken immediately before addition of PDPH, at a significant molar excess over the available free thiol groups, and periodically thereafter. The samples are immediately centrifuged and the supernatant absorbance at 343nm measured. Measurements are corrected for both the absorbance of the PDPH itself and the residual turbidity from unsedimented particles and the extent of reaction calculated using an extinction coefficient of 8080M$^{-1}$.cm$^{-1}$ for the released 2-thiopyridine. These data are used to determine optimum reaction conditions for coupling of PDPH to each particle type.

16.3 Reaction with EMCH

Aliquots of both types of labelled rHA particles are buffered at around pH7 by addition of phosphate buffer.

Samples are taken immediately before addition of EMCH, at a significant molar excess over the available free thiol groups, and periodically thereafter. The samples are immediately centrifuged and the pellets washed thoroughly to remove the excess EMCH prior to free thiol assay. These data are used to determine optimum reaction conditions for coupling of EMCH to each particle type.

Example 17

Preparation of antibody/Gd-labelled rHA particles

Based on the previous developments of individual steps in the synthesis (Examples.11-16), this method describes the preparation of a targeted agent, in which the particle (rHA) is labelled at high levels with DTPA followed by Gd and then coupled to an antibody, making it suitable for use as a targeted MRI contrast agent.

17.1 Synthesis of Gd-labelled rHA particles with and without cross-linker

Gd-labelled rHA particles and Gd-labelled rHA particles + cross-linker (PDPH or EMCH) are synthesised essentially as described in Example 16. The particles are then washed thoroughly by centrifugation and resuspension to remove excess reactants.

17.2 Synthesis of periodate-oxidised antibody with and without cross-linker

Periodate-oxidised antibody is synthesised by incubation of the antibody with $KIO_4$, PD10 chromatography and centrifugal concentration, essentially as described in Example 7. Coupling of PDPH cross-linker to the periodate-oxidised antibody and subsequent PD10 chromatography and centrifugal concentration are also performed essentially as described in Example 7. Coupling of EMCH cross-linker may be performed similarly.

17.3 Synthesis of antibody / Gd-labelled rHA particles

Antibody/Gd-labelled rHA particles may be produced by incubating any one of the following three combinations of reactants.

1. (Gd-labelled rHA particles + cross-linker) + (Periodate-oxidised antibody)
2. (Gd-labelled rHA particles) + (Periodate-oxidised antibody + cross-linker)
3. (Gd-labelled rHA particles) + (Periodate-oxidised antibody) + (Cross-linker) Whichever synthetic route is used, the resulting particles are washed thoroughly by centrifugation and resuspension to remove excess reactants.

Example 18

Preparation of antibody / DTPA-labelled rHA particles

This method describes the preparation of a targeted agent, in which the particle (rHA) is labelled at high levels with DTPA but no metal ion and then coupled to an antibody, making it suitable for loading with an appropriate radioactive metal for use as a targeted nuclear imaging or therapeutic agent.

18.1 Synthesis of DTPA-labelled rHA particles with and without cross-linker

DTPA-labelled rHA particles and DTPA-labelled rHA particles + cross-linker (PDPH or EMCH) are synthesised essentially as described in Example 16. The particles are then washed thoroughly by centrifugation and resuspension to remove excess reactants.

18.2 Synthesis of periodate-oxidised antibody with and without cross-linker

Periodate-oxidised antibody is synthesised by incubation of the antibody with $KIO_4$, PD10 chromatography and centrifugal concentration, essentially as described in Example 7. Coupling of PDPH cross-linker to the periodate-oxidised antibody and subsequent PD10 chromatography and centrifugal concentration are also performed essentially as described in Example 7. Coupling of EMCH cross-linker may be performed similarly.

18.3 Synthesis of antibody/Gd-labelled rHA particles

Antibody/DTPA-labelled rHA particles may be produced by incubating any one of the following three combinations of reactants.

1. (DTPA -labelled rHA particles + cross-linker) + (Periodate-oxidised antibody)
2. (DTPA -labelled rHA particles) + (Periodate-oxidised antibody + cross-linker)
3. (DTPA -labelled rHA particles) + (Periodate-oxidised antibody) + (Cross-linker) Whichever synthetic route is used, the resulting particles are washed thoroughly by centrifugation and resuspension to remove excess reactants.

**Claims**

1. A conjugate for use in medical imaging, which conjugate comprises a carrier in the form of a protein molecule or a particle formed from protein molecules, the carrier being bound to a contrast agent, or a precursor thereof, and to a targeting moiety having an affinity with a specific locus within the body,
   wherein the carrier molecule is coupled to the targeting moiety by a heterobifunctional cross-linking agent having one reactivity that is specific to functional groups present on the carrier and absent on the targeting moiety, and another reactivity that is specific to functional groups present on the targeting moiety and absent from the carrier.

2. A conjugate as claimed in Claim 1, which is for use in magnetic resonance imaging.

3. A conjugate as claimed in Claim 2, wherein the contrast agent comprises paramagnetic metal ions.

4. A conjugate as claimed in Claim 3, wherein the metal ions include $Gd^{3+}$ ions.

5. A conjugate as claimed in Claim 3 or Claim 4, wherein the metal ions are coupled to the carrier via a chelating moiety that is covalently bound to the carrier.

6. A conjugate as claimed in Claim 1, which is for use in nuclear imaging.

7. A conjugate as claimed in Claim 6, wherein the contrast agent comprises radioactive metal ions.

8. A conjugate as claimed in Claim 7, wherein the radioactive metal is selected from the group consisting of $^{99m}Tc$, $^{201}Tl$ and $^{111}In$.

9. A conjugate as claimed in Claim 7 or Claim 8, wherein the metal is coupled to the carrier via a chelating moiety that is covalently bound to the carrier.

10. A conjugate as claimed in Claim 5 or Claim 9, wherein the chelating moiety comprises carboxyl groups, or derivatives thereof, that react with amine groups in the carrier to form amide bonds.

11. A conjugate as claimed in Claim 10, wherein the chelating moiety is an acetic acid derivative of a compound comprising multiple amine groups.

12. A conjugate as claimed in Claim 11, wherein the chelating moiety is selected from the group consisting of ethylenediamine tetraacetic acid, diethylenetriamine pentaacetic acid, and derivatives thereof.

13. A conjugate for the delivery of a metal to the body, which conjugate comprises a carrier in the form of a protein molecule or a particle formed from protein molecules, the carrier being coupled via a chelating agent to said metal and conjugated with a targeting moiety having an affinity with a specific locus within the body, wherein the carrier molecule is coupled to the targeting moiety by a heterobifunctional cross-linking agent having one reactivity that is specific to functional groups present on the carrier and absent on the targeting moiety, and another reactivity that is specific to functional groups present on the targeting moiety and absent from the carrier.

14. A conjugate as claimed in Claim 5 or any one of Claims 9 to 13, wherein between 10 and 100 chelating moieties are coupled to each protein molecule.

15. A conjugate as claimed in Claim 14, wherein between 20 and 60 chelating moieties are coupled to each protein molecule.

16. A conjugate as claimed in Claim 1, which is for use in X-ray imaging.

17. A conjugate as claimed in Claim 16, wherein the contrast agent is an iodine compound.

18. A conjugate as claimed in any preceding claim, wherein the protein molecule is a single, complete or substantially complete, protein molecule.

19. A conjugate as claimed in any one of Claims 1 to 17, wherein the protein molecule is an oligomer of conjugated complete or substantially complete protein molecules.

20. A conjugate as claimed in Claim 19, wherein the oligomer comprises from two to twenty discrete protein molecules, more preferably up to ten, or up to five, discrete protein molecules.

21. A conjugate as claimed in any preceding claim, wherein the protein is derived from humans, or is identical, or substantially so, in structure to protein of human origin.

22. A conjugate as claimed in any preceding claim, wherein the protein is an albumin.

23. A conjugate as claimed in Claim 22, wherein the albumin is human serum albumin.

24. A conjugate as claimed in Claim 23, wherein the albumin is recombinant human serum albumin.

25. A conjugate as claimed in any preceding claim, which is soluble and
wherein the carrier comprises a protein molecule.

26. A conjugate as claimed in any one of Claims 1 to 24, wherein the carrier is in the form of a particle formed from protein molecules.

27. A conjugate as claimed in any preceding claim, wherein the targeting moiety is selected from-the group consisting of antibodies, other proteins and peptides.

28. A conjugate as claimed in Claim 27, wherein the targeting moiety is an antibody.

29. A conjugate as claimed in Claim 28, wherein the antibody is a monoclonal antibody.

30. A conjugate as claimed in any preceding claim, wherein the cross-linking agent comprises groups that are reactive to -SH groups on the carrier.

31. A conjugate as claimed in Claim 30, wherein the cross-linking agent comprises a group selected from the group consisting of maleimide, 2-pyridyldithio, haloacetate, haloacetamide, aziridine, acryloyl/vinyl, 4-pyridyldithio and 2-nitrobenzoate-5-dithio.

32. A conjugate as claimed in any preceding claim, wherein the cross-linking agent comprises groups that are reactive to carbohydrate moieties, or derivatives thereof, present on the targeting moiety.

33. A conjugate as claimed in Claim 32, wherein the cross-linking agent comprises a hydrazide group.

34. A conjugate as claimed in any one of Claims 30 to 33, wherein the cross-linking agent is selected from the group consisting of:

maleimidocaproic acid hydrazide;
3-(2-pyridyldithio)-propionyl hydrazide;
maleimidopropionic acid hydrazide;
N-(K-maleimidoundecanoic acid) hydrazide; and
4-(4-N-maleimidophenyl)butyric acid hydrazide.

**35.** A conjugate as claimed in Claim 25, wherein more than one carrier is coupled to the targeting moiety.

**36.** A conjugate as claimed in Claim 35, comprising from 2 to 10 carriers per targeting moiety.

**37.** A conjugate as claimed in Claim 36, comprising from 2 to 5 carriers per targeting moiety.

**38.** A conjugate as claimed in any preceding claim, wherein the targeting moiety is an antibody and is coupled to the carrier via a coupling site in the constant region of the antibody.

**39.** A conjugate as claimed in any preceding claim, in which at least 90%, more preferably at least 95%, and most preferably at least 99% of the agents for delivery to the body (or precursors thereof) present in the conjugate are covalently bound to the carrier.

**40.** A conjugate as claimed in any preceding claim, that has a binding activity of the targeting moiety in the conjugate, as measured in a competitive binding assay, of at least 50%, more preferably at least 60%, 70%, 80% or at least 90%, that of the free targeting moiety.

**41.** A formulation comprising a conjugate according to any preceding claim, in admixture with a pharmaceutically acceptable liquid medium.

**42.** A formulation as claimed in Claim 41, wherein the liquid medium is aqueous.

**43.** A method for the preparation of a targeted conjugate of an agent for delivery to the body with a carrier, which method comprises

(a) reacting the agent for delivery to the body, or a precursor thereof, with the carrier to-form an intermediate conjugate, and thereafter
(b) (i) reacting the intermediate conjugate with a heterobifunctional cross-linking agent to activate the intermediate conjugate and then reacting the activated intermediate conjugate with a targeting moiety, or (ii) reacting the intermediate conjugate with a targeting moiety that has been activated by reaction with a heterobifunctional cross-linking agent, or (iii) causing the intermediate conjugate, a heterobifunctional cross-linking agent and a targeting moiety to react simultaneously together,

wherein the cross-linking agent has one functionality that is specific for reaction with groups present on the carrier and absent from the targeting moiety, and a second functionality that is specific for reaction with groups present on the targeting moiety and absent from the carrier.

**44.** A method as claimed in Claim 43, wherein the carrier material is proteinaceous.

**45.** A method as claimed in Claim 44, wherein the carrier material is an albumin.

**46.** A method as claimed in Claim 45, wherein the albumin is human serum albumin.

**47.** A method as claimed in Claim 46, wherein the albumin is recombinant human serum albumin.

**48.** A method as claimed in any one of Claims 43 to 47, wherein the targeting moiety is an antibody.

**49.** A method as claimed in any one of Claims 43 to 48, wherein the cross-linking agent comprises a group that is reactive with sulphydryl groups.

**50.** A method as claimed in Claim 49, wherein the cross-linking agent comprises a group selected from the group consisting of maleimide, 2-pyridyldithio, haloacetate, haloacetamide, aziridine, acryloyl/vinyl, 4-pyridyldithio and 2-nitrobenzoate-5-dithio.

**51.** A method as claimed in any one of Claims 43 to 48, wherein the cross-linking agent comprises a group that is reactive with carbohydrate moieties or derivatives thereof.

**52.** A method as claimed in Claim 51, wherein the cross-linking agent comprises a hydrazide group.

**53.** A method as claimed in any one of Claims 43 to 52, wherein the agent for delivery to the body has, or is coupled to the carrier via an intermediate compound or moiety that contains, carboxyl groups.

**54.** A method as claimed in Claim 49, wherein step (b) is preceded by unblocking of sulphydryl groups present on the carrier.

**55.** A method as claimed in any one of Claims 43 to 54, wherein step (a) involves reacting the carrier with a chelating agent and the method further comprises formation of a chelate between the chelating agent bound to the carrier material and metal ions added to the reaction mixture, wherein the pH of the reaction mixture is maintained between 5.0 and 6.5 during addition of said metal ions.

**56.** A method as claimed in any one of Claims 43 to 55, wherein the carrier comprises a soluble protein molecule.

**57.** A method as claimed in any one of Claims 43 to 55, wherein the carrier is a particle, and the method comprises the preliminary step of forming the particle from particle-forming material.

**58.** A method as claimed in Claim 57, wherein the particle is formed by a method that comprises the steps of

i) forming a suspension of the particle-forming material; and
ii) spray-drying said suspension.

**59.** A method as claimed in Claim 58, wherein the suspension of particle-forming material is formed by dissolving the particle-forming material in a solvent, and then adding a non-solvent for the particle-forming material, so as to bring about precipitation of the particle-forming material.

**60.** A method as claimed in any of Claims 43 to 59, which is for the preparation of a conjugate as claimed in any one of Claims 1 to 40.

**61.** A conjugate prepared by the method of any one of Claims 43 to 60.

**62.** The use of a conjugate according to any one of Claims 1 to 40 or Claim 61 in the manufacture of a formulation for enhancing the contrast of an image to be obtained by a medical imaging technique.

**Patentansprüche**

**1.** Ein Konjugat zur Verwendung in medizinischen Bild gebenden Verfahren, das einen Träger in Form eines Proteinmoleküls oder einer aus Proteinmolekülen gebildeten Partikel umfasst, wobei der Träger an ein Kontrastmittel, oder einen Präkursor davon, und an einen Targeting-Anteil, der eine Affinität zu einem spezifischen Ort innerhalb des Körpers hat, gebunden ist, wobei das Trägermolekül durch ein heterobifunktionales Vernetzungsmittel, aufweisend eine Reaktivität, die für funktionelle Gruppen, die auf dem Träger vorhanden und auf dem Targeting-Anteil nicht vorhanden sind, spezifisch ist, und eine andere Reaktivität, die für funktionelle Gruppen, die auf dem Targeting-Anteil vorhanden und auf dem Träger nicht vorhanden sind, spezifisch ist, an den Targeting-Anteil gekoppelt ist.

**2.** Ein Konjugat nach Anspruch 1, das der Verwendung in der Magnetresonanztomografie dient.

**3.** Ein Konjugat nach Anspruch 2, wobei das Kontrastmittel paramagnetische Metallionen umfasst.

**4.** Ein Konjugat nach Anspruch 3, wobei die Metallionen $Gd^{3+}$-Ionen einschließen.

**5.** Ein Konjugat nach Anspruch 3 oder Anspruch 4, wobei die Metallionen über einen Chelat bildenden Anteil, der kovalent an den Träger gebunden ist, an den Träger gekoppelt sind.

**6.** Ein Konjugat nach Anspruch 1, das der Verwendung in nuklearen Bild gebenden Verfahren dient.

**7.** Ein Konjugat nach Anspruch 6, wobei das Kontrastmittel radioaktive Metallionen umfasst.

**8.** Ein Konjugat nach Anspruch 7, wobei das radioaktive Metall aus der Gruppe ausgewählt ist, die aus $^{99m}Tc$, $^{201}Tl$

und $^{111}$In besteht.

9. Ein Konjugat nach Anspruch 7 oder Anspruch 8, wobei das Metall über einen Chelat bildenden Anteil, der kovalent an den Träger gebunden ist, an den Träger gekoppelt ist.

10. Ein Konjugat nach Anspruch 5 oder Anspruch 9, wobei der Chelat bildende Anteil Carboxyl-Gruppen oder Derivate davon umfasst, die mit Amingruppen in dem Träger reagieren, um Amidbindungen zu bilden.

11. Ein Konjugat nach Anspruch 10, wobei der Chelat bildende Anteil ein Essigsäurederivat einer Verbindung ist, die mehrere Amingruppen umfasst.

12. Ein Konjugat nach Anspruch 11, wobei der Chelat bildende Anteil aus der Gruppe ausgewählt ist, die aus Ethylendiamintetraessigsäure, Diethylentriaminpentaessigsäure und Derivaten davon besteht.

13. Ein Konjugat zum Einbringen eines Metalls in den Körper, das einen Träger in Form eines Proteinmoleküls oder einer aus Proteinmolekülen gebildeten Partikel umfasst, wobei der Träger über ein Chelat bildendes Mittel an besagtes Metall gekoppelt ist und mit einem Targeting-Anteil konjugiert ist, der eine Affinität zu einem spezifischen Ort innerhalb des Körpers hat, wobei das Trägermolekül durch ein heterobifunktionales Vernetzungsmittel, aufweisend eine Reaktivität, die für funktionelle Gruppen, die auf dem Träger vorhanden und auf dem Targeting-Anteil nicht vorhanden sind, spezifisch ist, und eine andere Reaktivität, die für funktionelle Gruppen, die auf dem Targeting-Anteil vorhanden und auf dem Träger nicht vorhanden sind, spezifisch ist, an den Targeting-Anteil gekoppelt ist.

14. Ein Konjugat nach Anspruch 5 oder einem der Ansprüche 9 bis 13, wobei zwischen 10 und 100 Chelat bildende Anteile an jedes Proteinmolekül gekoppelt sind.

15. Ein Konjugat nach Anspruch 14, wobei zwischen 20 und 60 Chelat bildende Anteile an jedes Proteinmolekül gekoppelt sind.

16. Ein Konjugat nach Anspruch 1, das der Verwendung in Bild gebenden Röntgenverfahren dient.

17. Ein Konjugat nach Anspruch 16, wobei das Kontrastmittel eine Iodverbindung ist.

18. Ein Konjugat nach einem der vorangehenden Ansprüche, wobei das Proteinmolekül ein einziges, vollständiges oder im Wesentlichen vollständiges, Proteinmolekül ist.

19. Ein Konjugat nach einem der Ansprüche 1 bis 17, wobei das Proteinmolekül ein Oligomer aus konjugierten vollständigen oder im Wesentlichen vollständigen Proteinmolekülen ist.

20. Ein Konjugat nach Anspruch 19, wobei das Oligomer zwei bis zwanzig einzelne Proteinmoleküle, bevorzugter bis zu zehn, oder bis zu fünf, einzelne Proteinmoleküle umfasst.

21. Ein Konjugat nach einem der vorangehenden Ansprüche, wobei das Protein von Menschen stammt oder in seiner Struktur mit Protein menschlichen Ursprungs identisch oder im Wesentlichen identisch ist.

22. Ein Konjugat nach einem der vorangehenden Ansprüche, wobei das Protein ein Albumin ist.

23. Ein Konjugat nach Anspruch 22, wobei das Albumin humanes Serumalbumin ist.

24. Ein Konjugat nach Anspruch 23, wobei das Albumin rekombinantes humanes Serumalbumin ist.

25. Ein Konjugat nach einem der vorangehenden Ansprüche, das löslich ist und wobei der Träger ein Proteinmolekül umfasst.

26. Ein Konjugat nach einem der Ansprüche 1 bis 24, wobei der Träger die Form einer aus Proteinmolekülen gebildeten Partikel hat.

27. Ein Konjugat nach einem der vorangehenden Ansprüche, wobei der Targeting-Anteil aus der Gruppe ausgewählt ist, die aus Antikörpern, anderen Proteinen und Peptiden besteht.

**28.** Ein Konjugat nach Anspruch 27, wobei der Targeting-Anteil ein Antikörper ist.

**29.** Ein Konjugat nach Anspruch 28, wobei der Antikörper ein monoklonaler Antikörper ist.

**30.** Ein Konjugat nach einem der vorangehenden Ansprüche, wobei das Vernetzungsmittel Gruppen umfasst, die in Bezug auf-SH-Gruppen auf dem Träger reaktiv sind.

**31.** Ein Konjugat nach Anspruch 30, wobei das Vernetzungsmittel eine Gruppe umfasst, die aus der Gruppe ausgewählt ist, die aus Maleimid, 2-Pyridyldithio, Haloacetat, Haloacetamid, Aziridin, Acryloyl/Vinyl, 4-Pyridyldithio und 2-Nitrobenzoat-5-dithio besteht.

**32.** Ein Konjugat nach einem der vorangehenden Ansprüche, wobei das Vernetzungsmittel Gruppen umfasst, die in Bezug auf Kohlenhydrat-Anteile, oder Derivate davon, die auf dem Targeting-Anteil vorhanden sind, reaktiv sind.

**33.** Ein Konjugat nach Anspruch 32, wobei das Vernetzungsmittel eine HydrazidGruppe umfasst.

**34.** Ein Konjugat nach einem der Ansprüche 30 bis 33, wobei das Vernetzungsmittel aus der Gruppe ausgewählt ist, die aus
Maleimidocapronsäurehydrazid,
3-(2-Pyridyldithio)propionylhydrazid,
Maleimidopropionsäurehydrazid,
N-(K-Maleimidoundecansäure)hydrazid und
4-(4-N-Maleimidophenyl)buttersäurehydrazid
besteht.

**35.** Ein Konjugat nach Anspruch 25, wobei mehr als ein Träger an den Targeting-Anteil gekoppelt ist.

**36.** Ein Konjugat nach Anspruch 35, umfassend 2 bis 10 Träger pro Targeting-Anteil.

**37.** Ein Konjugat nach Anspruch 36, umfassend 2 bis 5 Träger pro Targeting-Anteil.

**38.** Ein Konjugat nach einem der vorangehenden Ansprüche, wobei der Targeting-Anteil ein Antikörper ist und über eine Kopplungsstelle in der konstanten Region des Antikörpers an den Träger gekoppelt ist.

**39.** Ein Konjugat nach einem der vorangehenden Ansprüche, bei dem mindestens 90 %, bevorzugter mindestens 95 % und am meisten bevorzugt mindestens 99 % der Mittel zum Einbringen in den Körper (oder Präkursoren davon), die in dem Konjugat vorhanden sind, kovalent an den Träger gebunden sind.

**40.** Ein Konjugat nach einem der vorangehenden Ansprüche, das eine Bindungsaktivität des Targeting-Anteiles in dem Konjugat, gemessen in einem kompetitiven Bindungsassay, von mindestens 50 %, bevorzugter mindestens 60 %, 70 %, 80 % oder mindestens 90 %, der Bindungsaktivität des freien Targeting-Anteiles hat.

**41.** Eine Formulierung, umfassend ein Konjugat nach einem der vorangehenden Ansprüche in Mischung mit einem pharmazeutisch akzeptablen flüssigen Medium.

**42.** Eine Formulierung nach Anspruch 41, wobei das flüssige Medium wässerig ist.

**43.** Ein Verfahren zur Darstellung eines Zielkonjugates eines Mittels zum Einbringen in den Körper mit einem Träger, wobei das Verfahren umfasst:

(a) Reagieren lassen des Mittels zum Einbringen in den Körper, oder eines Präkursors davon, mit dem Träger, um ein intermediäres Konjugat zu bilden, und anschließend
(b) (i) Reagieren lassen des intermediären Konjugates mit einem heterobifunktionalen Vernetzungsmittel, um das intermediäre Konjugat zu aktivieren, und anschließendes Reagieren lassen des aktivierten intermediären Konjugates mit einem Targeting-Anteil oder (ii) Reagieren lassen des intermediären Konjugates mit einem Targeting-Anteil, der durch Reaktion mit einem heterobifunktionalen Vernetzungsmittel aktiviert worden ist, oder (iii) Bewirken, dass das intermediäre Konjugat, ein heterobifunktionales Vernetzungsmittel und ein Targeting-Anteil gleichzeitig zusammen reagieren,

wobei das Vernetzungsmittel eine Funktionalität hat, die für die Reaktion mit Gruppen, die auf dem Träger vorhanden und auf dem Targeting-Anteil nicht vorhanden sind, spezifisch ist, und eine zweite Funktionalität hat, die für die Reaktion mit Gruppen, die auf dem Targeting-Anteil vorhanden und auf dem Träger nicht vorhanden sind, spezifisch ist.

44. Ein Verfahren nach Anspruch 43, wobei das Trägermaterial proteinartig ist.

45. Ein Verfahren nach Anspruch 44, wobei das Trägermaterial ein Albumin ist.

46. Ein Verfahren nach Anspruch 45, wobei das Albumin humanes Serumalbumin ist.

47. Ein Verfahren nach Anspruch 46, wobei das Albumin rekombinantes humanes Serumalbumin ist.

48. Ein Verfahren nach einem der Ansprüche 43 bis 47, wobei der Targeting-Anteil ein Antikörper ist.

49. Ein Verfahren nach einem der Ansprüche 43 bis 48, wobei das Vernetzungsmittel eine Gruppe umfasst, die in Bezug auf Sulfhydryl-Gruppen reaktiv ist.

50. Ein Verfahren nach Anspruch 49, wobei das Vernetzungsmittel eine Gruppe umfasst, die aus der Gruppe ausgewählt ist, die aus Maleimid, 2-Pyridyldithio, Haloacetat, Haloacetamid, Aziridin, Acryloyl/Vinyl, 4-Pyridyldithio und 2-Nitrobenzoat-5-dithio besteht.

51. Ein Verfahren nach einem der Ansprüche 43 bis 48, wobei das Vernetzungsmittel eine Gruppe umfasst, die in Bezug auf Kohlenhydrat-Anteile oder Derivate davon reaktiv ist.

52. Ein Verfahren nach Anspruch 51, wobei das Vernetzungsmittel eine HydrazidGruppe umfasst.

53. Ein Verfahren nach einem der Ansprüche 43 bis 52, wobei das Mittel zum Einbringen in den Körper Carboxyl-Gruppen hat oder über eine/n intermediäre/n Verbindung oder Anteil, die bzw. der Carboxyl-Gruppen enthält, an den Träger gekoppelt ist.

54. Ein Verfahren nach Anspruch 49, wobei dem Schritt (b) die Entblockung von auf dem Träger vorhandenen Sulfhydryl-Gruppen vorausgeht.

55. Ein Verfahren nach einem der Ansprüche 43 bis 54, wobei der Schritt (a) das Reagieren lassen des Trägers mit einem Chelat bildenden Mittel einschließt und das Verfahren ferner die Bildung eines Chelates zwischen dem an das Trägermaterial gebundenen Chelat bildenden Mittel und dem Reaktionsgemisch beigemischten Metallionen umfasst, wobei während der Beimischung besagter Metallionen das pH des Reaktionsgemisches zwischen 5,0 und 6,5 gehalten wird.

56. Ein Verfahren nach einem der Ansprüche 43 bis 55, wobei der Träger ein lösliches Proteinmolekül umfasst.

57. Ein Verfahren nach einem der Ansprüche 43 bis 55, wobei der Träger eine Partikel ist und das Verfahren den vorbereitenden Schritt der Bildung der Partikel aus Partikel bildendem Material umfasst.

58. Ein Verfahren nach Anspruch 57, wobei die Partikel durch ein Verfahren gebildet wird, das die folgenden Schritte umfasst:

  i) Bilden einer Suspension des Partikel bildenden Materials und
  ii) Sprühtrocknen besagter Suspension.

59. Ein Verfahren nach Anspruch 58, wobei die Suspension des Partikel bildenden Materials gebildet wird, indem das Partikel bildende Material in einem Lösungsmittel gelöst wird und dann ein Fällungsmittel für das Partikel bildende Material beigemischt wird, um die Fällung des Partikel bildenden Materials zu bewirken.

60. Ein Verfahren nach einem der Ansprüche 43 bis 59, das der Darstellung eines Konjugates nach einem der Ansprüche 1 bis 40 dient.

**61.** Ein Konjugat, dargestellt durch das Verfahren nach einem der Ansprüche 43 bis 60.

**62.** Die Verwendung eines Konjugates nach einem der Ansprüche 1 bis 40 oder nach Anspruch 61 bei der Herstellung einer Formulierung zur Verstärkung des Kontrastes eines durch ein medizinisches Bild gebendes Verfahren zu erlangenden Bildes.

**Revendications**

**1.** Conjugué pour une utilisation dans l'imagerie médicale, lequel conjugué comprend un véhicule sous la forme d'une molécule de protéine ou d'une particule formée à partir de molécules de protéine, le véhicule étant lié à un agent de contraste, ou un précurseur de celui-ci, et à une fraction de ciblage ayant une affinité avec un locus spécifique dans le corps, où la molécule de véhicule est couplée à la fraction de ciblage par un agent de réticulation hétérobifonctionnel ayant une réactivité qui est spécifique aux groupes fonctionnels présents sur le véhicule et absents sur la fraction de ciblage, et une autre réactivité qui est spécifique aux groupes fonctionnels présents sur la fraction de ciblage et absents du véhicule.

**2.** Conjugué selon la revendication 1, qui est destiné à une utilisation dans l'imagerie par résonance magnétique.

**3.** Conjugué selon la revendication 2, dans lequel l'agent de contraste comprend des ions métalliques paramagnétiques.

**4.** Conjugué selon la revendication 3, dans lequel les ions métalliques comprennent les ions $Gd^{3+}$.

**5.** Conjugué selon la revendication 3 ou la revendication 4, dans lequel les ions métalliques sont couplés au véhicule par le biais d'une fraction de chélation qui est liée par covalence au véhicule.

**6.** Conjugué selon la revendication 1, qui est destiné à une utilisation dans l'imagerie nucléaire.

**7.** Conjugué selon la revendication 6, dans lequel l'agent de contraste comprend des ions métalliques radioactifs.

**8.** Conjugué selon la revendication 7, dans lequel le métal radioactif est choisi dans le groupe constitué de $^{99m}Tc$, $^{201}Tl$ et $^{111}In$.

**9.** Conjugué selon la revendication 7 ou la revendication 8, dans lequel le métal est couplé au véhicule par le biais d'une fraction de chélation qui est liée par covalence au véhicule.

**10.** Conjugué selon la revendication 5 ou la revendication 9, dans lequel la fraction de chélation comprend des groupes carboxyle, ou des dérivés de ceux-ci, qui réagissent avec des groupes amine dans le véhicule pour former des liaisons amide.

**11.** Conjugué selon la revendication 10, dans lequel la fraction de chélation est un dérivé d'acide acétique d'un composé comprenant de multiples groupes amine.

**12.** Conjugué selon la revendication 11, dans lequel la fraction de chélation est choisie dans le groupe constitué de l'acide éthylènediamine tétraacétique, de l'acide diéthylènetriamine pentaacétique et de dérivés de ceux-ci.

**13.** Conjugué pour la distribution d'un métal au corps, lequel conjugué comprend un véhicule sous la forme d'une molécule de protéine ou d'une particule formée à partir de molécules de protéine, le véhicule étant couplé par le biais d'un agent de chélation audit métal et conjugué avec une fraction de ciblage ayant une affinité avec un locus spécifique dans le corps, où la molécule de véhicule est couplée à la fraction de ciblage par un agent de réticulation hétérobifonctionnel ayant une réactivité qui est spécifique aux groupes fonctionnels présents sur le véhicule et absents sur la fraction de ciblage, et une autre réactivité qui est spécifique aux groupes fonctionnels présents sur la fraction de ciblage et absents du véhicule.

**14.** Conjugué selon la revendication 5 ou l'une quelconque des revendications 9 à 13, dans lequel entre 10 et 100 fractions de chélation sont couplées à chaque molécule de protéine.

**15.** Conjugué selon la revendication 14, dans lequel entre 20 et 60 fractions de chélation sont couplées à chaque molécule de protéine.

**16.** Conjugué selon la revendication 1, qui est destiné à une utilisation dans l'imagerie par rayons X.

**17.** Conjugué selon la revendication 16, dans lequel l'agent de contraste est un composé d'iode.

**18.** Conjugué selon l'une quelconque des revendications précédentes, dans lequel la molécule de protéine est une seule molécule de protéine complète ou sensiblement complète.

**19.** Conjugué selon l'une quelconque des revendications 1 à 17, dans lequel la molécule de protéine est un oligomère de molécules de protéine conjuguées complètes ou sensiblement complètes.

**20.** Conjugué selon la revendication 19, dans lequel l'oligomère comprend deux à vingt molécules de protéine discrètes, de manière davantage préférée jusqu'à dix, ou jusqu'à cinq, molécules de protéine discrètes.

**21.** Conjugué selon l'une quelconque des revendications précédentes, dans lequel la protéine est dérivée d'êtres humains, ou est identique, ou sensiblement identique, en termes de structure à la protéine d'origine humaine.

**22.** Conjugué selon l'une quelconque des revendications précédentes, dans lequel la protéine est une albumine.

**23.** Conjugué selon la revendication 22, dans lequel l'albumine est une albumine de sérum humain.

**24.** Conjugué selon la revendication 23, dans lequel l'albumine est une albumine de sérum humain recombinante.

**25.** Conjugué selon l'une quelconque des revendications précédentes, qui est soluble et dans lequel le véhicule comprend une molécule de protéine.

**26.** Conjugué selon l'une quelconque des revendications 1 à 24, dans lequel le véhicule est sous la forme d'une particule formée à partir de molécules de protéine.

**27.** Conjugué selon l'une quelconque des revendications précédentes, dans lequel la fraction de ciblage est choisie dans le groupe constitué d'anticorps, d'autres protéines et de peptides.

**28.** Conjugué selon la revendication 27, dans lequel la fraction de ciblage est un anticorps.

**29.** Conjugué selon la revendication 28, dans lequel l'anticorps est un anticorps monoclonal.

**30.** Conjugué selon l'une quelconque des revendications précédentes, dans lequel l'agent de réticulation comprend des groupes qui sont réactifs aux groupes -SH sur le véhicule.

**31.** Conjugué selon la revendication 30, dans lequel l'agent de réticulation comprend un groupe choisi dans le groupe constitué du maléimide, du 2-pyridyldithio, de l'halogénoacétate, de l'halogénoacétamide, de l'aziridine, de l'acryloyle/vinyle, du 4-pyridyldithio et du 2-nitrobenzoate-5-dithio.

**32.** Conjugué selon l'une quelconque des revendications précédentes, dans lequel l'agent de réticulation comprend des groupes qui sont réactifs aux fractions glucide, ou dérivés de celles-ci, présentes sur la fraction de ciblage.

**33.** Conjugué selon la revendication 32, dans lequel l'agent de réticulation comprend un groupe hydrazide.

**34.** Conjugué selon l'une quelconque des revendications 30 à 33, dans lequel l'agent de réticulation est choisi dans le groupe constitué de :

l'hydrazide d'acide maléimidocaproïque ;
l'hydrazide de 3-(2-pyridyldithio)-propionyle ;
l'hydrazide d'acide maléimidopropionique ;
l'hydrazide de N-(κ-acide maléimidoundécanoïque) ; et
l'hydrazide d'acide 4-(4-N-maléimidophényl)butyrique.

**35.** Conjugué selon la revendication 25, dans lequel plus d'un véhicule est couplé à la fraction de ciblage.

**36.** Conjugué selon la revendication 35, comprenant 2 à 10 véhicules par fraction de ciblage.

**37.** Conjugué selon la revendication 36, comprenant 2 à 5 véhicules par fraction de ciblage.

**38.** Conjugué selon l'une quelconque des revendications précédentes, dans lequel la fraction de ciblage est un anticorps et est couplée au véhicule par le biais d'un site de couplage dans la région constante de l'anticorps.

**39.** Conjugué selon l'une quelconque des revendications précédentes, dans lequel au moins 90 %, de manière davantage préférée au moins 95 %, et de manière préférée entre toutes au moins 99 % des agents pour distribution au corps (ou précurseurs de ceux-ci) présents dans le conjugué sont liés par covalence au véhicule.

**40.** Conjugué selon l'une quelconque des revendications précédentes, qui a une activité de liaison de la fraction de ciblage dans le conjugué, comme mesuré dans un dosage de liaison compétitif, d'au moins 50 %, de manière davantage préférée d'au moins 60 %, 70 %, 80 % ou d'au moins 90 %, de celle de la fraction de ciblage libre.

**41.** Formulation comprenant un conjugué selon l'une quelconque des revendications précédentes, en mélange avec un milieu liquide pharmaceutiquement acceptable.

**42.** Formulation selon la revendication 41, dans laquelle le milieu liquide est aqueux.

**43.** Procédé de préparation d'un conjugué cible d'un agent pour distribution au corps avec un véhicule, lequel procédé comprend

(a) la réaction de l'agent pour distribution au corps, ou un précurseur de celui-ci, avec le véhicule pour former un conjugué intermédiaire, et par la suite
(b) (i) la réaction du conjugué intermédiaire avec un agent de réticulation hétérobifonctionnel pour activer le conjugué intermédiaire, puis la réaction du conjugué intermédiaire activé avec une fraction de ciblage, ou (ii) la réaction du conjugué intermédiaire avec une fraction de ciblage qui a été activée par réaction avec un agent de réticulation hétérobifonctionnel, ou (iii) le fait d'amener le conjugué intermédiaire, un agent de réticulation hétérobifonctionnel et une fraction de ciblage à réagir simultanément ensemble,

dans lequel l'agent de réticulation a une fonctionnalité qui est spécifique de la réaction avec des groupes présents sur le véhicule et absents de la fraction de ciblage, et une seconde fonctionnalité qui est spécifique de la réaction avec des groupes présents sur la fraction de ciblage et absents du véhicule.

**44.** Procédé selon la revendication 43, dans lequel le matériau de véhicule est protéique.

**45.** Procédé selon la revendication 44, dans lequel le matériau de véhicule est une albumine.

**46.** Procédé selon la revendication 45, dans lequel l'albumine est une albumine de sérum humain.

**47.** Procédé selon la revendication 46, dans lequel l'albumine est une albumine de sérum humain recombinante.

**48.** Procédé selon l'une quelconque des revendications 43 à 47, dans lequel la fraction de ciblage est un anticorps.

**49.** Procédé selon l'une quelconque des revendications 43 à 48, dans lequel l'agent de réticulation comprend un groupe qui est réactif avec des groupes sulfhydryle.

**50.** Procédé selon la revendication 49, dans lequel l'agent de réticulation comprend un groupe choisi dans le groupe constitué du maléimide, du 2-pyridyldithio, de l'halogénoacétate, de l'halogénoacétamide, de l'aziridine, de l'acryloyle/vinyle, du 4-pyridyldithio et du 2-nitrobenzoate-5-dithio.

**51.** Procédé selon l'une quelconque des revendications 43 à 48, dans lequel l'agent de réticulation comprend un groupe qui est réactif avec des fractions glucide ou des dérivés de celles-ci.

**52.** Procédé selon la revendication 51, dans lequel l'agent de réticulation comprend un groupe hydrazide.

**53.** Procédé selon l'une quelconque des revendications 43 à 52, dans lequel l'agent pour distribution au corps a, ou est couplé au véhicule par le biais d'un composé ou d'une fraction intermédiaire qui contient des groupes carboxyle.

**54.** Procédé selon la revendication 49, dans lequel l'étape (b) est précédée par le déblocage de groupes sulfhydryle présents sur le véhicule.

**55.** Procédé selon l'une quelconque des revendications 43 à 54, dans lequel l'étape (a) implique la réaction du véhicule avec un agent de chélation et le procédé comprend en outre la formation d'un chélate entre l'agent de chélation lié au matériau de véhicule et les ions métalliques ajoutés au mélange de réaction, où le pH du mélange de réaction est maintenu entre 5,0 et 6,5 pendant l'addition desdits ions métalliques.

**56.** Procédé selon l'une quelconque des revendications 43 à 55, dans lequel le véhicule comprend une molécule de protéine soluble.

**57.** Procédé selon l'une quelconque des revendications 43 à 55, dans lequel le véhicule est une particule, et le procédé comprend l'étape préliminaire de formation de la particule à partir d'un matériau de formation de particule.

**58.** Procédé selon la revendication 57, dans lequel la particule est formée par un procédé qui comprend les étapes consistant à

   i) former une suspension du matériau de formation de particule ; et
   ii) sécher par pulvérisation ladite suspension.

**59.** Procédé selon la revendication 58, dans lequel la suspension du matériau de formation de particule est formée en dissolvant le matériau de formation de particule dans un solvant, puis en ajoutant un non-solvant pour le matériau de formation de particule, de sorte à entraîner la précipitation du matériau de formation de particule.

**60.** Procédé selon l'une quelconque des revendications 43 à 59, qui est destiné à la préparation d'un conjugué selon l'une quelconque des revendications 1 à 40.

**61.** Conjugué préparé par le procédé selon l'une quelconque des revendications 43 à 60.

**62.** Utilisation d'un conjugué selon l'une quelconque des revendications 1 à 40 ou la revendication 61 dans la fabrication d'une formulation pour optimiser le contraste d'une image à obtenir par une technique d'imagerie médicale.

Figure 1

Figure 2

## Figure 3

## Figure 4

### a) Sephadex G25 chromatography

### b) Sephadex G25 chromatography

c) Sephadex G50 chromatography (large scale)

Figure 5

Figure 6

Figure 7

## Figure 8

### a) αCD45-PDPH-rHA-DTPA-Gd

log (fluorescent intensity)

### b) Unlabelled αCD45

log (fluorescent intensity)

Figure 9

## Figure 10a

## Figure 10b

## Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9412218 A
- US 4794170 A
- WO 9001900 A
- US 5929044 A

- WO 03015756 A
- WO 9637515 A
- WO 0044772 A

**Non-patent literature cited in the description**

- **BONIFACE et al.** *J Nucl Med,* 1989, vol. 30, 683-691
- **PAIK et al.** *J Nucl Med,* 1987, vol. 24, 1158-1163
- **UNGER et al.** *Investigative Radiol,* 1985, vol. 20 (7), 693-700
- **SHAHBAZI-GAHROUEI et al.** *Aust Phys and Eng Sci in Med,* 2002, vol. 25 (1), 31-38
- **MATSUMURA et al.** *Acta neurochirurgica,* 1994, vol. 60, 356-358
- **CURTET et al.** *Intl J Cancer,* 1988, vol. 2, 126-132
- **GERHARD et al.** *MRM,* 1994, vol. 32, 622-628
- **LAUFFER et al.** *Magnetic Resonance Imaging,* 1985, vol. 3 (1), 11-16
- **PIMM et al.** *Eur J Nucl Med,* 1992, vol. 19, 449-452
- **GOHR-ROSENTHAL et al.** *Invest Radiol,* 1993, vol. 28, 789-795
- **MANABE et al.** *Biochemica at Biophysica Acta,* 1986, vol. 883 (3), 460-467
- **PIMM et al.** Human tumour xenografts in anticancer drug development. Springer Verlag, 1988, 95-98
- **HARTUNG et al.** *Clin Cancer Res,* 1999, vol. 5 (4), 753-759

- **CARIDE.** *Critical reviews in therapeutic drug carrier systems,* 1985, vol. 1 (2), 121-153
- **TILCOCK.** *Adv Drug Deliv Reviews,* 1999, vol. 37, 33-51
- **KUNIMASA et al.** *Chem and Pharm Bulletin (Japan),* 1992, vol. 40 (9), 2565-2567
- **WINTER et al.** *Magnetic Resonance in Medicine,* 2003, vol. 50 (2), 411-416
- **SUWA et al.** *Intl J Cancer,* 1998, vol. 75 (4), 626-634
- **SUZUKI et al.** *Brain Tumour Pathology,* 1996, vol. 13 (2), 127-132
- **ARTEMOV et al.** *Magnetic Resonance in Medicine,* 2003, vol. 49 (3), 403-408
- **YU et al.** *Magnetic Resonance in Medicine,* 2000, vol. 44 (6), 867-872
- **ANDERSON et al.** *Magnetic Resonance in Medicine,* 2000, vol. 44 (3), 433-439
- **SAMTEN.** *Chinese J Microbiol and Immunol,* 1996, vol. 16, 54-57
- **KLIBANOV.** *Adv Drug Deliv Reviews,* 1999, vol. 37, 139-157
- **KERR.** *EXS (Switzerland),* 1992, vol. 61, 339-345
- **MACGILLIVRAY et al.** Molecular and Cellular Iron Transport. Marcel Dekker, Inc, 2002, 41
- **MASON et al.** *Biochemistry,* 1993, vol. 32, 5472